(19) Europäisches Patentamt
European Patent Office
Office européen des brevets

(11) EP 2 182 933 B1

(12) **EUROPÄISCHE PATENTSCHRIFT**

(45) Veröffentlichungstag und Bekanntmachung des Hinweises auf die Patenterteilung:
**07.09.2016 Patentblatt 2016/36**

(21) Anmeldenummer: **08786245.4**

(22) Anmeldetag: **18.07.2008**

(51) Int Cl.:
***A61K 9/72*** *(2006.01)* ***A61K 31/137*** *(2006.01)*
***A61K 31/46*** *(2006.01)*

(86) Internationale Anmeldenummer:
**PCT/EP2008/059465**

(87) Internationale Veröffentlichungsnummer:
**WO 2009/013244 (29.01.2009 Gazette 2009/05)**

(54) **NEUE PULVERFÖRMIGE ARZNEIMITTEL ENTHALTEND TIOTROPIUM UND SALMETEROL SOWIE LAKTOSE ALS HILFSSTOFF**

NOVEL MEDICAMENT IN POWDER FORM COMPRISING TIOTROPIUM AND SALMETEROL, AND LACTOSE AS EXCIPIENT

NOUVEAUX MÉDICAMENTS SOUS FORME DE POUDRE CONTENANT DU TIOTROPIUM ET DU SALMÉTÉROL AINSI QUE DU LACTOSE COMME EXCIPIENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.07.2007 DE 102007034157**
**09.05.2008 US 51933 P**

(43) Veröffentlichungstag der Anmeldung:
**12.05.2010 Patentblatt 2010/19**

(73) Patentinhaber: **Boehringer Ingelheim International GmbH**
**55216 Ingelheim am Rhein (DE)**

(72) Erfinder:
- **WALZ, Michael**
  **55216 Ingelheim am Rhein (DE)**
- **OSSADNIK, Stephanie**
  **55216 Ingelheim am Rhein (DE)**
- **TRUNK, Michael**
  **55216 Ingelheim am Rhein (DE)**
- **KREHER, Christoph**
  **55216 Ingelheim am Rhein (DE)**

(74) Vertreter: **Simon, Elke Anna Maria et al**
**Boehringer Ingelheim GmbH**
**Binger Strasse 173**
**55216 Ingelheim am Rhein (DE)**

(56) Entgegenhaltungen:
**EP-A- 1 514 549** **WO-A-02/11803**
**WO-A-2004/058233** **WO-A-2007/042822**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 182 933 B1

**Beschreibung**

Die Erfindung betrifft stabile Arzneimittelzusammensetzungen zur inhalativen Anwendung, die eine Kombination von Tiotropiumsalzen **1** mit Salmeterolsalzen **2** als Zubereitung mit Laktose beinhalten. Des Weiteren betrifft die Erfindung Verfahren zu deren Herstellung sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

## Hintergrund der Erfindung

Sowohl Tiotropiumsalze **1** als auch Salmeterolsalze **2** sind aus dem Stand der Technik bekannt und gelangen beide bei der Therapie von Atemwegserkrankungen zur Anwendung.

Tiotropiumbromid ist aus der Europäischen Patentanmeldung EP 418 716 A1 bekannt und weist die folgende chemische Struktur auf:

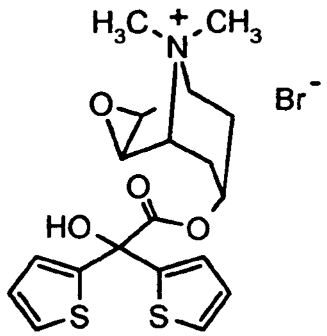

Auch die Kombination von Tiotropiumsalzen **1** und Salmeterolsalzen **2** ist im Stand der Technik bekannt. Sie wird neben anderen Arzneimittelkombination von langwirksamen Betamimetika mit langwirksamen Anticholinergika in der WO 00/69468 offenbart.

Pulverförmige Zubereitungen von Arzneimitteln zur Inhalation, welche die Kombination von Salmeterolxinafoat und Tiotropiumsalzen beschreiben, sind in WO2004/0582 offenbart. Die dort offenbarten Formulierungen sind durch ein hohes Maß an Homogenität und Gleichförmigkeit gekennzeichnet.

Pulverinhalativa werden beispielsweise als Inhalationspulver in geeignete Kapseln abgefüllt und mittels Pulverinhalatoren appliziert werden. Ebenso sind weitere Systeme, in denen die zu applizierende Pulvermenge vordosiert ist (z.B. Blister) als auch Multidose Pulversysteme bekannt. Übliche Herstellverfahren von Pulverinhalativa, z.B. in Form von Kapseln zur Inhalation, werden beispielsweise in der DE-A-179 22 07 beschrieben. Ein weiterer bedeutender Aspekt bei Pulverinhalativa ist, dass bei der inhalativen Applikation des Wirkstoffes nur Teilchen einer bestimmten aerodynamischen Größe in das Zielorgan Lunge gelangen. Die Teilchengröße dieser lungengängigen Partikel (inhalierbarer Anteil) liegt im Bereich weniger $\mu m$, typischerweise zwischen 1 und $10\mu m$, vorzugsweise zwischen 1 und $6\mu m$. Solche Partikel werden üblicherweise durch Mikronisierung (Luftstrahlmahlung) erzeugt.

Verpackungseinheiten für pharmazeutische Produkte, die die Aufrechterhaltung spezifischer Eigenschaften von inhalierbaren Arzneimittelzubereitungen gewährleisten sollen, sind in der Literatur in allgemeiner Art beschrieben. Die pharmazeutischen Wirkstoffe, z.B. in Form von Kapseln oder Tabletten, sind dabei häufig in Blisterkarten verpackt, wobei die Kavitäten der Blisterkarten den Wirkstoff vor Umwelteinflüssen von außen schützen. Zum Schutz vor Feuchteeinflüssen können derartige Verpackungseinheiten zusätzlich Trockenmittel enthalten. Eine derartige Verpackungseinheit wird beispielsweise in Form einer Faltschachtel mit Blisterkarten durch die EP 0479282 A1 offenbart.

Beim Einsatz konventioneller Trockenmittel, wie beispielsweise Silicagel oder Molekularsieb (Zeolithe), stellt sich eine nicht kontrollierbare Restfeuchte in der Umgebung ein, beispielsweise in einem Schlauchbeutel aus einer Aluminium-Verbundfolie oder in einer HD-PE-Flasche, die Bestandteil einer Verpackungseinheit für pharmazeutische Produkte sein können. Diese Feuchte ist abhängig von der Art des Trockenmittels, der bereits vorhandenen Wasserbeladung des Trockenmittels, der Menge an Trockenmittel und der vorhandenen Wasserquellen, beispielsweise der Feuchtegehalt der Packstoffe, des Arzneimittels und der eingeschlossenen Luft oder aber auch das während der Lagerung eindringende Wasser.

In der Regel wird ein deutlicher Trockenmittelüberschuss in die Verpackung gegeben, um auf jeden Fall eine zuverlässige Trocknungswirkung zu erzielen. Eine solche Verpackungseinheit, welche ein Trockenmittel zur vollständigen Entfernung der Umgebungsfeuchte enthält, wird beispielsweise von der WO 2004/105727 A2 offenbart. Bei der Verwendung von unkonditioniertem Trockenmittel hat dies aber zur Folge, dass eine Restfeuchte von weniger als 2 %

rel. Feuchte erreicht werden kann..

Überraschenderweise wurde gefunden, dass die aus dem Stand der Technik genannten Vorgehensweisen nicht dazu geeignet sind, Inhalationspulver bereitzustellen, die neben Tiotropiumsalzen und Salmeterol den Hilfsstoff Laktose enthalten und die durch ein ausreichendes Maß an Stabilität gekennzeichnet sind.

Im Rahmen der vorliegenden Erfindungen werden unter stabilen Inhalationspulvern solche Inhalationspulver verstanden, deren Eigenschaften auch über einen längeren Zeitraum unverändert erhalten bleiben. Inhalationspulver verändern dann ihre Eigenschaften nicht, wenn sowohl die chemische Stabilität der einzelnen Komponenten in der Pulvermischung als auch deren physikalische bzw. physikochemische Stabilität gegeben ist. Dies setzt auch voraus, dass die Komponenten der Pulvermischung hinsichtlich ihrer polymorphen und morphologischen Eigenschaften unverändert bleiben.

Es ist Aufgabe der vorliegenden Erfindung, pulverförmige Arzneimittelzubereitungen zur Inhalation bereit zu stellen, die neben einem Tiotropiumsalz **1** und einem Salmeterolsalz **2** als Hilfsstoff Laktose enthalten und die durch ein hohes Maß an Stabilität gekennzeichnet ist.

Es ist ferner Aufgabe der vorliegenden Erfindung vorstehend genannte pulverförmige Arzneimittelzusammensetzungen bereitzustellen, deren Verwendung die Inhalation der Wirkstoffe mit hohem inhalierbarem Anteil erlaubt.

Der inhalierbare Anteil stellt dabei die Menge an inhalierbaren Wirkstoffpartikeln (< 5μm) dar, wie sie auf Basis der Pharm. Eur. 2.9.18 (European Pharmacopoeia, 6th edition 2008, Apparatus D-Andersen Cascade Impactor) bzw. USP30-NF25 <601> bestimmbar ist. Der inhalierbare Anteil wird im Rahmen der vorliegenden Erfindung auch als FPD (Fine Particle Dose) bezeichnet.

Es ist ferner Aufgabe der vorliegenden Erfindung vorstehend genannte pulverförmige Arzneimittelzusammensetzungen bereitzustellen, die durch einen hohen Fine Particle Fraction (FPF) gekennzeichnet sind. Dabei stellt die FPF die relative FPD bezogen auf die nominale Dosis pro Applikation dar. Die FPF ist somit dadurch erhältlich, indem die FPD gemäß obigen Angaben bestimmt wird, und diese zur nominalen Dosis in Bezug gesetzt wird (Angabe in [%]).

Es ist insbesondere Aufgabe der vorliegenden Erfindung vorstehend genannte pulverförmige Arzneimittelzusammensetzungen bereitzustellen, die dadurch gekennzeichnet sind, dass sich die FPF auch bei Lagerung über einen längeren Zeitraum, bevorzugt 18 Monate (Lagerbedingung gemäß ICH-Guideline 25°C und 60% relative Feuchte) durch eine hohe Konstanz auszeichnet.

Darüberhinaus ist eine weitere Aufgabe der vorliegenden Erfindung, Inhalationspulver bereit zu stellen, deren inhalierbarer Anteil weitestgehend unabhängig von der Flussrate bei der Ausbringung ist (Ausbringung in Anlehnung an die Bestimmung der FPD, jedoch bei variiertem Fluss: 20L/min, 30 L/min, 40L/min, 60 L/min)

Ebenso stellt sich die Aufgabe, Herstellverfahren für erfindungsgemäße Arzneimittelzubereitungen zur Verfügung zu stellen.

Detaillierte Beschreibung der Erfindung

Überraschenderweise wurde gefunden, dass die eingangs genannten Aufgaben durch erfindungsgemäße, pulverförmige, Zubereitungen für die Inhalation (Inhalationspulver) gelöst werden, die neben einem Tiotropiumsalz **1** ferner ein Salmeterolsalz **2** und den Hilfsstoff Laktose enthalten und die durch einen $a_w$-Wert zwischen 0.1 und 0.4 gekennzeichnet sind.

Dementsprechend betrifft die vorliegende Erfindung Verfahren zur Bereitstellung von Inhalationspulver enthaltend Laktose, ein Salmeterolsalz und ein Tiotropiumsalz dadurch gekennzeichnet, dass das Inhalationspulver einer Feuchte ausgesetzt wird, so dass sich über dem Inhalationspulver ein aw-Wert bei 25°C zwischen 0.1 und 0.4 im Gleichgewicht einstellt, dadurch gekennzeichnet, dass die Inhalationspulver

- mind. 4 Stunden bei einer rel. Feuchte von 18 bis 27% und 16°C bis 28°C konditioniert werden
- darauf folgend die vordosierten Inhalationspulver in einer Umgebungsfeuchte von 20 bis 30% und einer Temperatur von 23 bis 28°C verpackt werden.

Unter dem $a_w$-Wert (auch Wasseraktivität oder Activity of Water) versteht man hierbei das Maß für frei verfügbares Wasser in einem Material. Dieser ist definiert als Quotient des Wasserdampfdruckes (p) über einem Material, hier die Arzneimittelzubereitung, zu dem Wasserdampfdruck über reinem Wasser (p0) bei einer bestimmten Temperatur, hier 25°C, im Gleichgewicht:

$$a_W\text{-Wert} = p/p0.$$

Erfindungsgemäße Arzneimittelzubereitungen sind ferner dadurch gekennzeichnet, dass diese einen $a_w$-Wert zwischen 0.10 und 0.40, bevorzugt zwischen 0.15 und 0.40 und besonders bevorzugt zwischen 0.15 und 0.35 aufweisen.

Erfindungsgemäß ist es hierbei möglich, dass die Arzneimittelzusammensetzungen mittels eines Konditionierschrittes in das Gleichgewicht mit der direkt über dem Produkt befindlichen relativen Feuchte gebracht werden, so dass ein $a_w$-Wert zwischen 0.10 und 0.40, bevorzugt zwischen 0.15 und 0.40 und besonders bevorzugt zwischen 0.15 und 0.35 vorliegt.

Der für die erfindungsgemäßen Arzneimittelzubereitungen charakteristische aw-Wert kennzeichnet hierbei die Arzneimittelzubereitungen, wie sie nach Herstellung der Bulkware, sowie nach deren Verpackung als auch während ihrer Laufzeit bis zur Entnahme des Arzneimittels aus dem Packmittel (im Rahmen der bestimmungsgemäßen Anwendung als Arzneimittel) vorliegen.

Im Rahmen der vorliegenden Erfindung ist das Salmeterolsalz **2** in Form eines Säureadditionssalzes enthalten. Besonders bevorzugte Inhalationspulver enthalten als Salmeterolsalz **2** Salmeterolxinafoat (ie: (R,S)-4-Hydroxy-$\alpha$1-[[[6-(4-phenylbutoxy)hexyl]amino]methyl]-1,3-benzendimethanol 1-hydroxy-2-naphthalencarboxylat). Unter Salmeterol **2'** ist die freie Salmeterolbase zu verstehen.

Im Rahmen der vorliegenden Erfindung werden unter Tiotropiumsalzen **1** Salze verstanden, die durch von dem pharmakologisch wirksamen Kation Tiotropium **1'** gebildet werden. Im Rahmen der vorliegenden Patentanmeldung ist eine explizite Bezugnahme auf das Kation Tiotropium durch Verwendung der Bezeichnung **1'** erkennbar. Unter Tiotropium **1'** ist das freie Ammoniumkation zu verstehen. Wird im Rahmen der vorliegenden Erfindung die Bezeichnung **1** verwendet, so ist dies als Bezugnahme auf Tiotropium in Kombination mit einem entsprechenden Gegenion zu verstehen. Als Gegenion (Anion) kommen bevorzugt Chlorid, Bromid, Iodid, Methansulfonat oder para-Toluolsulfonat in Betracht. Von diesen Anionen ist das Bromid bevorzugt.

Bevorzugt werden zur Herstellung der erfindungsgemäßen Tiotropium-haltigen Inhalationspulver die Hydrate des Tiotropiumbromids eingesetzt. Besonders bevorzugt wird dabei das aus der WO 02/30928 bekannte kristalline Tiotropiumbromid-monohydrat verwendet. Dieses kristalline Tiotropiumbromid-Monohydrat ist gekennzeichnet durch ein bei der thermischen Analyse mittels DSC auftretendes endothermes Maximum bei 230 $\pm$ 5°C bei einer Heizrate von 10K/min. Ferner ist es dadurch gekennzeichnet daß es im IR-Spektrum unter anderem bei den Wellenzahlen 3570, 3410, 3105, 1730, 1260, 1035 und 720 $cm^{-1}$ Banden aufweist. Schließlich weist dieses kristalline Tiotropiumbromid-monohydrat, wie mittels Einkristallröntgenstrukturanalyse bestimmt, eine einfache monoklinische Zelle mit folgenden Dimensionen auf: a = 18.0774 Å, b = 11.9711 Å, c = 9.9321 Å, $\beta$ = 102.691°, V = 2096.96 $Å^3$.

Die oben genannten Wirkstoffe finden erfindungsgemäß ihren Einsatz in Form ihrer Mikronisate. Zur Durchführung des Prozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinanderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels Speisegas unter spezifischen Drücken (Speisedruck). Im Rahmen der vorliegenden Erfindung hat sich ein Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts kann dabei in einer Förderrate von etwa 3-65 g/min, bevorzugt mit 5 - 35 g/min, besonders bevorzugt mit etwa 10-30 g/min erfolgen.

Die erfindungsgemäßen Arzneimittelzubereitungen sind weiterhin dadurch gekennzeichnet, dass diese als pharmazeutisch verträglichen Hilfsstoff Laktose enthalten. Als besonders bevorzugt im Sinne der Erfindung gelangt als Hilfsstoff Lactosemonohydrat zur Anwendung.

Besonders bevorzugt werden Hilfsstoffe verwendet, die eine mittlere Teilchengröße von 15 bis 65$\mu$m, in besonders bevorzugten Inhalationspulvern ist der Hilfsstoff durch eine mittlere Teilchengröße von 20 bis 47 $\mu$m, besonders bevorzugt von 27 bis 45 $\mu$m gekennzeichnet. Dabei wird unter der mittleren Teilchengröße im hier verwendeten Sinne der 50 % - Wert aus der Volumenverteilung, gemessen mit einem Laserdiffraktometer nach der Trockendispersionsmethode verstanden.

Besonders bevorzugt werden ferner solche Hilfsstoffe verwendet, die einen 10%-Feinanteil von 1 bis 8 $\mu$m aufweisen. Dabei ist unter dem 10 % - Feinanteil im hier verwendeten Sinne der 10 % - Wert aus der mit einem Laserdiffraktometer gemessenen Volumenverteilung zu verstehen. Mit anderen Worten steht im Sinne der vorliegenden Erfindung der 10%-Feinanteil-Wert für die Teilchengröße, unterhalb derer 10% der Teilchenmenge liegt (bezogen auf Volumenverteilung). Ferner sind insbesondere solche Inhalationspulver besonders bevorzugt, in denen der 10%-Feinanteil des Hilfsstoffes etwa 2 bis 7 $\mu$m, bevorzugt etwa 3 bis 6 $\mu$m beträgt.

Erfindungsgemäß bevorzugt sind ferner solche Inhalationspulver, in denen der Hilfsstoff eine spezifische Oberfläche zwischen 0,2 und 1,5 $m^2$/g, bevorzugt zwischen 0,3 und 1,2 $m^2$/g, besonders bevorzugt 0,4 und 1,0 $m^2$/g besitzt.

Für die erfindungsgemäßen Pulverformulierungen werden bevorzugt Laktose hoher Kristallinität verwendet. Diese Kristallinität kann anhand der beim Lösen des Hilfsstoffs freiwerdenden Enthalpie (Lösungsenthalpie) beurteilt werden. Im Falle des erfindungsgemäß besonders bevorzugt zum Einsatz gelangenden Hilfsstoffs Laktosemonohydrat, wird vorzugsweise Laktose verwendet, die durch eine Lösungsenthalpie von ≥ 45 J/g, bevorzugt von ≥ 50 J/g, besonders bevorzugt von ≥ 52 J/g gekennzeichnet ist.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8% , bevorzugt < 6% , besonders bevorzugt < 4% (relative Standardabweichung bezogen auf Einzeldosen-Gehaltsbestimmungen).

Gegebenenfalls kann es hilfreich sein, alternativ zu den vorstehend genannten Hilfsstoffen Hilfsstoffgemische zu verwenden, die aus einem Gemisch von gröberem Hilfsstoff mit einer mittleren Teilchengröße von 17 bis 50µm, bevorzugt von 20 bis 40µm, besonders bevorzugt 25 bis 35µm und feinerem Hilfsstoff mit einer mittleren Teilchengröße von 1 bis 8µm, bevorzugt von 2 bis 7µm, besonders bevorzugt 3 bis 6µm bestehen. Auch hier wird unter der mittleren Teilchengröße der 50%-Wert aus der Volumenverteilung gemessen mittels Laserbeugung nach der Trockendispersionsmethode verstanden.
Werden vorstehend genannte Hilfsstoffgemische verwendet, liegt der 10%-Feinanteil der gröberen Hilfsstoffkomponente bei etwa 2 bis 5 µm, bevorzugt etwa 3 bis 4 µm, und der der feineren Hilfsstoffkomponente bei etwa 0,5 bis 1,5 µm. Bevorzugt sind Inhalationspulver bei denen der Anteil von feinerem Hilfsstoff an der Gesamtformulierung 2 bis 10%, bevorzugt 3 bis 7%, besonders bevorzugt 4 bis 6% beträgt. Wird im Rahmen der vorliegenden Erfindung auf die Bezeichnung Hilfsstoffgemisch Bezug genommen, so ist hierbei stets eine Mischung zu verstehen, die durch Mischen zuvor klar defmierter Komponenten erhalten wurde. Entsprechend sind beispielsweise als Hilfsstoffgemisch aus gröberen und feineren Hilfsstoffanteilen nur solche Gemische zu verstehen, die durch Mischen einer gröberen Hilfsstoffkomponente mit einer feineren Hilfsstoffkomponente erhalten werden.

Dementsprechend sind erfindungsgemäße Arzneimittelzubereitungen derart zusammengesetzt, dass diese 3µg bis 1000µg, bevorzugt 5µg bis 500µg, besonders bevorzugt 10µg bis 250µg, ferner bevorzugt 15µg bis 150µg, erfindungsgemäß bevorzugt 20µg bis 100µg, im besonderen bevorzugt 25µg bis 50µg **2** pro Einmalgabe enthalten, wobei dieser Wirkstoff in einer definierten Menge von Laktose, bevorzugt Laktose Monohydrat, welche zwischen 5mg und 50 mg, z.B. 5 mg, 6 mg, 7 mg, 8 mg, 9mg, 10mg, 11 mg, 12mg, 13mg, 14mg, 15mg, 16mg, 17mg, 18mg, 19mg, 20mg, 21kg, 22mg, 23mg, 24mg, 25mg oder jeden sonstigen Wert zwischen 5mg und 50mg, homogen verteilt vorliegt. Darüber hinaus enthalten die erfindungsgemäßen Arzneimittelzubereitungen pro Einzeldosis 1µg bis 5000µg **1**, bevorzugt 2µg bis 2000µg **1**, besonders bevorzugt 3µg bis 1000µg **1**, ferner bevorzugt 4µg bis 500µg **1**, erfindungsgemäß bevorzugt 5µg bis 250µg **1**, darüber hinaus bevorzugt 6µg bis 100µg **1** und im besonderen darüber hinaus bevorzugt 7µg bis 25µg **1**.

Bevorzugt sind erfindungsgemäße Arzneimittelzubereitungen, die pro Einmalgabe die folgenden Mengen an **2**’ und **1**’ enthalten: **2**’ 12.5µg und **1**’ 15µg in 5 mg Laktose, **2**’ 25µg und **1**’ 15µg in 5 mg Laktose, **2**’ 50µg und **1**’ 15µg in 5 mg Laktose, **2**’ 100µg und **1**’ 15µg in 5 mg Laktose, **2**’ 12.5µg und **1’** 10µg in 5 mg Laktose, **2’** 25µg und **1’** 10µg in 5 mg Laktose, **2’** 50µg und **1’** 10µg in 5 mg Laktose, **2**’ 100µg und **1’** 10µg in 5 mg Laktose, **2’** 12.5µg und **1’** 7.5µg in 5 mg Laktose, **2’** 25µg und **1’** 7.5µg in 5 mg Laktose, **2**’ 50µg und **1’** 7.5µg in 5 mg Laktose, **2**’ 100µg und **1’** 7.5µg in 5 mg Laktose, **2’** 12.5µg und **1’** 5µg in 5 mg Laktose, **2’** 25µg und **1’** 5µg in 5 mg Laktose, **2**’ 50µg und **1’** 5µg in 5 mg Laktose, **2**’ 100µg und **1**’ 5µg in 5 mg Laktose, **2**’ 12.µg und **1**’ 3.8µg in 5 mg Laktose, **2**’ 25µg und **1**’ 3.µg in 5 mg Laktose, **2**’ 50µg und **1’** 3.8µg in 5 mg Laktose, **2**’ 100µg und **1**’ 3.8µg in 5 mg Laktose, **2**’ 12.5µg und **1’** 15µg in 10 mg Laktose, **2**’ 25µg und **1**’ 15µg in 10 mg Laktose, **2**’ 50µg und **1**’ 15µg in 10 mg Laktose, **2**’ 100µg und **1’** 015µg in 10 mg Laktose, **2**’ 12.5µg und **1**’ 10µg in 10 mg Laktose, **2**’ 25µg und **1**’ 10µg in 10 mg Laktose, **2**’ 50µg und **1**’ 10µg in 10 mg Laktose, **2**’ 100µg und **1**’ 10µg in 10 mg Laktose, **2**’ 12.5µg und **1**’ 7.5µg in 10 mg Laktose, **2**’ 25µg und **1**’ 7.5µg in 10 mg Laktose, **2**’ 50µg und **1**’ 7.5µg in 10 mg Laktose, **2**’ 100µg und **1**’ 7.5µg in 10 mg Laktose, **2**’ 12.5µg und **1**’ 5µg in 10 mg Laktose, **2**’ 25µg und **1**’ 5µg in 10 mg Laktose, **2**’ 50µg und **1**’ 5µg in 10 mg Laktose, **2**’ 100µg und **1**’ 5µg in 10 mg Laktose, **2**’ 12.5µg und **1**’ 3.8µg in 10 mg Laktose, **2**’ 25µg und **1’** 3.8µg in 10 mg Laktose, **2**’ 50µg und **1**’ 3.8µg in 10 mg Laktose, **2**’ 100µg und **1**’ 3.8µg in 10 mg Laktose, **2**’ 12.5µg und **1**’ 15µg in 15 mg Laktose, **2**’ 25µg und **1’** 15µg in 15 mg Laktose, **2**’ 50µg und **1’** 15µg in 15 mg Laktose, **2**’ 100µg und **1**’ 15µg in 15 mg Laktose, **2**’ 12.5µg und **1**’ 10µg in 15 mg Laktose, **2**’ 25µg und **1**’ 10µg in 15 mg Laktose, **2**’ 50µg und **1**’ 10µg in 15 mg Laktose, **2**’ 100µg und **1**’ 10µg in 15 mg Laktose, **2**’ 12.5µg und **1**’ 7.5µg in 15 mg Laktose, **2**’ 25µg und **1’** 7.5µg in 15 mg Laktose, **2**’ 50µg und **1’** 7.5µg in 15 mg Laktose, **2**’ 100µg und **1’** 7.5µg in 15 mg Laktose, **2**’ 12.5µg und **1**’ 5µg in 15 mg Laktose, **2**’ 25µg und **1**’ 5µg in 15 mg Laktose, **2**’ 50µg und **1**’ 5µg in 15 mg Laktose, **2**’ 100µg und **1’** 5µg in 15 mg Laktose, **2**’ 12.5µg und **1**’ 3.8µg in 15 mg Laktose, **2**’ 25µg und **1**’ 3.8µg in 15 mg Laktose, **2**’ 50µg und **1**’ 3.8µg in 15 mg Laktose, **2**’ 100µg und **1**’ 3.8µg in 15 mg Laktose, **2**’ 12.5µg und **1’** 15µg in 20 mg Laktose, **2’** 25µg und **1’** 15µg in 20 mg Laktose, **2**’ 50µg und **1’** 15µg in 20 mg Laktose, **2**’ 100µg und **1**’ 15µg in 20 mg Laktose, **2**’ 12.5µg und **1**’ 10µg in 20 mg Laktose, **2**’ 25µg und **1**’ 10µg in 20 mg Laktose, **2**’ 50µg und **1’** 10µg in 20 mg Laktose, **2**’ 100µg und **1**’ 10µg in 20 mg Laktose, **2**’ 12.5µg und **1**’ 7.5µg in 20 mg Laktose, **2**’ 25µg und **1**’ 7.5µg in 20 mg Laktose, **2**’ 50µg und **1**’ 7.5µg in 20 mg Laktose,

**2**’ 100μg und **1’** 7.5μg in 20 mg Laktose, **2**’ 12.5μg und **1**’ 5μg in 20 mg Laktose, **2**’ 25μg und **1**’ μg in 20 mg Laktose, **2**’ 50μg und **1**’ 5g in 20 mg Laktose, **2**’ 100μg und **1**’ 5μg in 20 mg Laktose, **2**’ 12.5μg und **1**’ 3.8μg in 20 mg Laktose, **2**’ 25μg und **1**’ 3.8μg in 20 mg Laktose, **2**’ 50μg und **1’** 3.8μg in 20 mg Laktose, **2**’ 100μg und **1**’ 3.8μg in 20 mg Laktose, **2**’ 12.5μg und **1**’ 15μg in 25 mg Laktose, **2**’ 25μg und **1’** 15μg in 25 mg Laktose, **2**’ 50μg und **1**’ 15μg in 25 mg Laktose, **2**’ 100μg und **1**’ 15μg in 25 mg Laktose, **2**’ 12.5μg und **1**’ 10μg in 25 mg Laktose, **2**’ 25μg und **1**’ 10μg in 25 mg Laktose, **2**’ 50μg und **1**’ 10μg in 25 mg Laktose, **2**’ 100μg und **1**’ 10μg in 25 mg Laktose, **2’** 12.5μg und **1**’ 7.5μg in 25 mg Laktose, **2**’ 25μg und **1**’ 7.5μg in 25 mg Laktose, **2**’ 50μg und **1**’ 7.5μg in 25 mg Laktose, **2**’ 100μg und **1**’ 7.5μg in 25 mg Laktose, **2**’ 12.5μg und **1**’ 5μg in 25 mg Laktose, **2**’ 25μg und **1**’ 5μg in 25 mg Laktose, **2**’ 50μg und **1**’ 5μg in 25 mg Laktose, **2**’ 100μg und **1**’ 5μg in 25 mg Laktose, **2**’ 12.5μg und **1**’ 3.8μg in 25 mg Laktose, **2**’ 25μg und **1**’ 3.8μg in 25 mg Laktose, **2**’ 50μg und **1**’ 3.8μg in 25 mg Laktose, **2**’ 100μg und **1**’ 3.8μg in 25 mg Laktose enthalten sind.

Der erfindungsgemäße Konditionierschritt zur Einstellung eines spezifischen aw-Wertes bedingt ein weiteres Anfeuchten oder ein Trocknen der Arzneimittelzubereitung in Abhängigkeit der absoluten Feuchte der Arzneimittelzubereitung, wobei im Sinn der Erfindung hierbei die abgefüllte Arzneimittelzubereitung, z.B. abgefüllt in Inhalationskapseln oder in Blisterkavitäten verstanden wird.

Erfindungsgemäß werden die Aufgaben bei dem Verfahren zur Bereitstellung der erfindungsgemäßen Arzneimittelzubereitungen dadurch gelöst, dass als besondere Ausführung der Erfindung gezielt der Feuchtegehalt in einer Verpackungseinheit eingestellt wird, in dem vor der Einbringung beispielweise eines Trockenmittels in die Verpackungseinheit das Trockenmittel im Rahmen eines zusätzlichen Konditionierungsschrittes einer definierten Feuchtigkeitsatmosphäre mit einer bestimmten Restfeuchte ausgesetzt wird.

Aufgrund dieser Maßnahmen kann über die Lagerdauer eines Arzneimittels die Feuchtigkeit innerhalb der Verpackungseinheit in einer definierten Bandbreite kontrolliert werden, d.h. es kann sowohl die Überschreitung eines oberen Grenzwertes als auch die Unterschreitung eines unteren Grenzwertes sicher vermieden werden. Dadurch wird das Arzneimittel vor negativen Auswirkungen einer zu hohen und einer zu niedrigen Feuchte geschützt. Stabilitätsanforderungen, insbesondere bei komplexen Wirkstoffkombinationen, können somit besser erfüllt werden. Mögliche Strukturveränderungen bei manchen Wirkstoffen, die zu einer veränderten und damit unerwünschten, pharmazeutischen Wirkung führen, werden vermieden.

Zur Darstellung der erfindungsgemäßen Arzneimittel ist es zunächst erforderlich, die Ausgangsmaterialien der erfindungsgemäßen Arzneimittelzubereitungen als homogene Pulvermischungen bereit zu stellen.
Nach Einwaage der Ausgangsmaterialien erfolgt die Herstellung der Pulvermischungen aus dem Hilfsstoff und dem Wirkstoff bzw. den Wirkstoffen unter Verwendung von im Stand der Technik bekannten Verfahren. Hierbei sei beispielsweise auf die Offenbarung der WO 02/30390 und WO2004/058233 verwiesen. Die erfindungsgemäßen Inhalationspulver sind dementsprechend beispielsweise gemäß der nachfolgend beschriebenen Vorgehensweise erhältlich. Bei den nachstehend beschriebenen Herstellverfahren werden die genannten Komponenten in den Gewichtsanteilen eingesetzt, wie sie in den zuvor beschriebenen Zusammensetzungen der Inhalationspulver beschrieben wurden. Alternativ können die homogenen Pulvermischungen auch mittels High-Shearmischer (Intensiv-Mischer, Diosna-Mischer) sowie Low-Shearmischer (Schrauben-Mischer, Rubergmischer) hergestellt werden. Dabei kann beispielsweise ca. 1/3 der Gesamtmenge des Hilfsstoffes (Trägermaterial) Laktose in den Mischbehälter vorgelegt werden. Die Zugabe erfolgt vorzugsweise über ein Sieb oder einen Siebgranulator mit einer Maschenweite von 0,1 bis 2 mm, besonders bevorzugt 0,3 bis 1 mm, höchst bevorzugt 0,3 bis 0,6 mm. Als Variante kann dabei der Sieb mit dem Hilfsstoff zwischengespült werden, sofern die Zugabe des Wirkstoffes bzw. der Wirkstoffe Portionsweise erfolgt. Nach der Zugabe des Wirkstoffes erfolgt ein Nachspülen des Siebes mit dem Hilfsstoff. Bevorzugt ist das abwechselnde, schichtweise Einsieben der verschiedenen Komponenten.

Sofern die in den oben beschriebenen Verfahren eingesetzten Wirkstoffe nicht bereits nach ihrer chemischen Herstellung in einer kristallinen Form erhältlich sind, die vorstehend genannte Teilchengrößen aufweist, können sie durch Mahlen in die Teilchengrößen überführt werden, die den vorstehend genannten Parametern genügen (sogenanntes Mikronisieren). Entsprechende Mikronisierverfahren sind aus dem Stand der Technik (z.B. WO2004/058233) bekannt. Besonders bewährt hat sich zur Mahlung der Salmeterolsalze **2** beispielsweise und ohne den Gegenstand der Erfindung darauf zu beschränken, als eine mögliche Ausführungsform einer Luftstrahlmühle das folgende Gerät: Chrispro Jet-Mill MC200, Mahlkammergröße = 200 mm mit Mahldüsen 2,25 mm, Firma Micro Macinazione SA, Via Cantonale, 6995 Molinazzo di Monteggio (CH). Dabei hat sich zur Mikronisierung der Salmeterolsalze **2** besonders bewährt, wenn der Mahldruck auf einen Wert zwischen etwa 2 und etwa 9 bar, bevorzugt zwischen etwa 3 und etwa 6 bar, besonders bevorzugt zwischen etwa 3,5 und etwa 4,5 bar eingestellt ist. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2,5 und etwa 9,5 bar, bevorzugt zwischen etwa 3,5 und etwa 6,5 bar, besonders bevorzugt zwischen etwa 4 und etwa 5 bar bewährt. Als Speisegas gelangt vorzugsweise ebenfalls ein Inertgas, besonders bevorzugt ebenfalls Stickstoff zur Anwendung. Die Zufuhr des Mahlguts (bevorzugt kristallines Salmeterolxinafoat) kann dabei in einer Förderrate von etwa 100 - 300 g/min, vorzugsweise mit etwa 150 - 250 g/min erfolgen.

Zur Durchführung des Prozesses können alternativ gängige Luftstrahl-Mühlen als auch Gegenstrahl-Mühlen

zum Einsatz gelangen. Bevorzugt wird die Mikronisierung dabei unter Feuchtigkeitsausschluss durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinanderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) zerkleinert.

Optional kann das so erhaltene Mahlgut anschließend unter den nachfolgend genannten spezifischen Bedingungen weiterverarbeitet werden. Hierzu wird das Mikronisat bei einer Temperatur von 15-40 °C, vorzugsweise 20 - 35 °C, besonders bevorzugt bei 25 - 30 °C Wasserdampf einer relativen Feuchte von wenigstens 40% ausgesetzt. Vorzugsweise wird die Feuchte auf einen Wert von 50 - 95% r.F., bevorzugt auf 60 - 90 % r.F., besonders bevorzugt auf 70 - 80 % r.F. eingestellt.

Unter relativer Feuchte (r.F.) wird hier der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mikronisat den genannten Raumbedingungen allerdings für etwa 12 bis etwa 48 Stunden, vorzugsweise etwa 18 bis etwa 36 Stunden, besonders bevorzugt etwa 20 bis etwa 28 Stunden ausgesetzt.

Das analog zu vorstehender Vorgehensweise erhältliche Mikronisat des Tiotropiumsalzes **1**, bevorzugt des Tiotropiumbromids - es sei hierbei auch auf WO2004/058233 verwiesen - weist eine mittlere Teilchengröße von zwischen 0,5 μm und 10 μm, bevorzugt zwischen 1 μm und 6 μm, besonders bevorzugt zwischen 2 μm und 5,0μm und $Q_{(5.8)}$ von größer 60%, bevorzugt größer 70 %, besonders bevorzugt größer 80% auf. Dabei bezeichnet der Kennwert $Q_{(5.8)}$ die Teilchenmenge der Partikel, die bezogen auf die Volumenverteilung der Partikel unterhalb von 5.8 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das erfindungsgemäß bevorzugt zum Einsatz gelangende Tiotropium-Mikronisat, das nach obigem Prozeß dargestellt wurde, sind spezifische Oberflächenwerte im Bereich zwischen 2 $m^2$/g und 5 $m^2$/g, im besonderen Maße Werte zwischen 2,5 $m^2$/g und 4,5 $m^2$/g und in besonders herausragendem Maße zwischen 3,0 $m^2$/g und 4,0 $m^2$/g.

Salmeterotsalz-Mikronisat **2** weist eine mittlere Teilchengröße von zwischen 0,5 μm und 10 μm, bevorzugt zwischen 1 μm und 6 μm, besonders bevorzugt zwischen 1,5 μm und 4,0μm und $Q_{(3,0)}$ von zwischen 50% und 90%, bevorzugt zwischen 60% und 80%, besonders bevorzugt zwischen 65% und 75% auf. Dabei bezeichnet der Kennwert $Q_{(3,0)}$ die Teilchenmenge der Partikel, die bezogen auf die Volumenverteilung der Partikel unterhalb von 3,0 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das erfindungsgemäße bevorzugt zum Einsatz gelangende Salmeterolsalz-Mikronisat, das nach obigem Prozeß dargestellt wurde, sind spezifische Oberflächenwerte im Bereich zwischen 5 $m^2$/g und 10 $m^2$/g, im besonderen Maße Werte zwischen 5,5 $m^2$/g und 9 $m^2$/g und in besonders herausragendem Maße zwischen 6,0 $m^2$/g und 8 $m^2$/g.

Als eine bevorzugte Variante kann das Herstellverfahren zur Bereitstellung eines Mikronisats vor Weiterverarbeitung desselben, d.h. vor Herstellung der Pulvermischung, einen weiteren Herstellschritt aufweisen. Hierbei werden die einzusetzenden mikronisierten Wirkstoffe gemäß Struktur **1** und / oder **2**, durch einen Ultraschallsieb mit einer Maschenweite von 80 μm oder 63 μm oder 40 μm oder 25 μm oder durch einen Sieb mit einer Mascheweite zwischen 25μm und 80μm gegeben.

Die erfindungsgemäßen Pulverformulierungen finden bevorzugt Anwendung als vordosierte Arzneimittelzubereitung. Hierzu können erfindungsgemäße Pulvermischungen nach im Stand der Technik bekanntem Verfahren vordosiert werden. Bevorzugt ist dabei die Abfüllung einer definierten Menge, welche zwischen 5mg und 50 mg, z.B. 5 mg, 6 mg, 7 mg, 8 mg, 9mg, 10mg, 11 mg, 12mg, 13mg, 14mg, 15mg, 16mg, 17mg, 18mg, 19mg, 20mg, 21mg, 22mg, 23mg, 24mg, 25mg oder jeden sonstigen Wert zwischen 5mg und 50mg betragen kann. Erfindungsgemäß bevorzugt ist dabei ein Dosisbehältnis in Form einer Kavität zur Aufnahme einer Pulvermenge, die als Einmaldosis verabreicht wird.

Überraschenderweise hat sich gezeigt, dass sich im Nachgang an die Abfüllung ein Äquilibrierschritt / Konditionierschritt als vorteilhaft erweist. Darunter versteht man erfindungsgemäß einen Prozessschritt, indem der Wassergehalt des Produktes derart verändert wird, dass dieses als Folge dieses Prozessschrittes einen aw-Wert zwischen 0.10 und 0.40, besonders bevorzugt zwischen 0.15 und 0.40 und ganz besonders bevorzugt zwischen 0.15 und 0.35 aufweist.

Technisch bedeutet dies, dass mittels des Konditionierschrittes dem Produkt entweder weiteres Wasser zugeführt oder abgeführt wird in Abhängigkeit des absoluten Wassergehaltes des Produktes. Hierbei hat sich gezeigt, dass mittels eines Einschritt-Prozesses die erfindungsgemäße Zielfeuchte unabhängig vom Ist-Zustandes des Produktes nach Abfüllung des Inhalationspulver erreicht werden kann, wenn das Produkt einer spezifischen relativen Feuchte ***RH*** bei einer Temperatur ***T*** über einen Zeitraum t ausgesetzt wird. Geeignete Prozessparameter sind, wenn der Prozess

bei ***RH*** = 15-35%, ***T*** = 15-30°C und ***t*** >4h, bevorzugt bei ***RH*** = 18-27%, ***T*** = 16-28°C und ***t*** >4h, besonders bevorzugt bei ***RH*** =19-26%, ***T*** = 17-27°C und ***t*** > 4h, ganz besonders bevorzugt bei ***RH*** = 20-25%, ***T*** = 19-23°C und ***t*** > 4h durchgeführt wird und dabei die erfindungsgemäßen Arzneimittezubereitungen, z.B. abgefüllt in Kapseln, in einem geschlossenem Behälter diesen Prozessbedingungen ausgesetzt sind. Dabei ist erfindungsgemäß darauf zu achten, dass die Prozessparameter ***RH*** und ***T*** während der Durchführung des Prozesses innerhalb des angegebenen Bereiches gehalten und nachgeführt werden. Unter relativer Feuchte (***RH***) wird hier der Quotient des Partialdruckes des Wasserdampfs und des Dampfdruckes des Wassers bei der betreffenden Temperatur verstanden. Es zeigt sich hierbei, dass eine Verlängerung der Prozesszeit t bei gleichzeitiger Einhaltung der möglichen Prozessparameter ***RH*** und ***T*** gemäß obigen Angaben auf ***t*** > 8h, ***t*** > 12h, ***t*** > 24h bzw. ***t*** > 48h für die Durchführung des Prozesses bevorzugt ist.

Mögliche Verfahren jedoch nicht im Sinne einer abschließenden Auflistung zur Durchführung obigen Prozessschrittes können derart ausgestaltet sein, dass erfindungsgemäße abgefüllte Arzneimittelzubereitungen in einem Container dem indirekten Kontakt, d.h. nicht Produkt-berührend, wobei ein Austausch der Feuchte über die Gasphase gegeben ist, einem vorkonditioniertem Trockenmittel ausgesetzt werden. Dabei ist ein unbegrenzter Gasaustausch zwischen Arzneimittelzubereitung und dem vorkonditioniertem Trockenmittel gegeben. In einem vorbereitenden Schritt ist hierbei das Trockenmittel mittels handelsüblicher Klimakammern (z.B. Weiss-Klimakammer, Firma Weiss Klimatechnik GmbH, 35447 Reiskirchen-Lindenstruth, Deutschland) in das Gleichgewicht gemäß den Vorgaben obiger Prozessparameter ***RH, T*** und ***t*** zu bringen. Zum Einsatz können hierbei handelsübliche Trockenmittel wie beispielsweise Silicagel, Molekularsieb (Zeolithe), Betonit kommen, wobei dem Silicalgel eine herausragende Bedeutung zukommt.
Alternativ ist es auch möglich, erfindungsgemäße abgefüllte Arzneimittelzubereitungen in einem Container mittels eines Gasstromes, der obigen Prozessparametern für ***RH, T*** und ***t*** entspricht, kontinuierlich zu durchströmen.
Desweiteren ist alternativ ein Verfahren möglich, bei dem das Produkt direkt, z.B. ausgebreitet auf Hordenblechen, den klimatischen Bedingungen gemäß obiger Prozessparameter in einer ausreichend regelbaren Klimakammer unter Einhaltung obiger Prozessparameter für ***RH, T*** und ***t*** ausgesetzt wird.

Erfindungsgemäß bevorzugt ist eine Vordosierung der Arzneimittelzubereitung in ein Dosisbehältnis, welches aus einem Material hergestellt ist, das zumindest an der Kontaktfläche zwischen dem Inhalationspulver ein Material aufweist, welches ausgewählt ist aus der Gruppe der synthetischen Kunststoffen. Ebenso sind erfindungsgemäß Dosisbehältnisse bevorzugt, welche aus einem Material ausgewählt sind, die durch ihre Eigenschaft, dass sie nicht-hygroskopisch sind, charakterisiert werden können. Darunter versteht man die Eigenschaft, Feuchtigkeit aus der Umgebung (meist in Form von Wasserdampf aus der Luftfeuchtigkeit) zu binden und bei Bedarf sehr schnell wieder abgeben zu können. Im Sinne der vorliegenden Erfindung ist ein Material dann als nicht-hygroskopisch anzusehen, wenn dieses weniger als 0,5% (w/w), bevorzugt weniger als 0,2% (w/w), ganz bevorzugt weniger als 0,1% (w/w) Wasser aufnehmen bzw. abgeben kann bei einer Temperatur von 25°C bezogen auf eine Gleichgewichts-Umgebungsfeuchte von 5% rel. Feuchte im Vergleich zu 75% rel. Feuchte. Entsprechende Messverfahren sind dem Fachmann bekannt, stellvertretend und beispielhaft für einen möglichen Messaufbau ist hier auf das DVS-System der Fa. Porotec GmbH (D-65719 Hofheim, Germany) verwiesen. Erfindungsgemäß ebenso bevorzugt sind Dosisbehältnisse, die aus einem Material hergestellt sind, welche dadurch gekennzeichnet sind, dass diese eine elektrische Leitfähigkeit $\sigma$ (sigma) aufweisen, welche kleiner als $10^{-5}$ S $cm^{-1}$, bevorzugt kleiner $10^{-10}$ S $cm^{-1}$ ist.

Als eine bevorzugte vordosierte Arzneimittelzubereitung sind befüllte Kapseln zu nennen, die die erfindungsgemäßen Inhalationspulver enthalten. Die Befüllung derselben erfolgt nach im Stand der Technik bekannten Verfahren der leeren Kapseln mit den erfindungsgemäßen Inhalationspulvern. Hierfür werden besonders bevorzugt solche Kapseln verwendet, deren Material ausgewählt ist aus der Gruppe der synthetischen Kunststoffe, besonders bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/$m^3$, bevorzugt von 940 - 980 kg/$m^3$, besonders bevorzugt von etwa 960 - 970 kg/$m^3$ (high-density Polyethylen) zur Anwendung. Erfindungsgemäß sind den Kapseln Pulverreservoire gleichgestellt, in welche die erfindungsgemäßen Arzneimittelzubereitungen Produkt-berührend abgefüllt sind. Darunter versteht man, dass erfindungsgemäße Pulverreservoire derart ausgestaltet sind, dass zumindest das die Arzneimittelzubereitung kontaktierende Material aus einem Material ausgewählt ist aus der Gruppe der synthetischen Kunststoffen.
Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genanntes erfindungsgemäßes Inhalationspulver enthalten. Diese Kapseln können etwa 1 bis 20mg, bevorzugt etwa 3 bis 15, besonders bevorzugt etwa 4 bis 12 mg Inhalationspulver enthalten. Erfindungsgemäß bevorzugte Formulierungen enthalten 4 bis 6 mg Inhalationspulver. Von erfindungsgemäß gleichrangiger Bedeutung sind Inhalationskapseln, die die

erfindungsgemäßen Formulierungen in einer Menge von 8 bis 12 mg, besonders bevorzugt 9 bis 11 mg enthalten.

Erfindungsgemäße vordosierte Arzneimittel sind zur Sicherstellung, dass das Produkt einem erfindungsgemäßen $a_w$-Wert entspricht, unter kontrollierten Prozessparametern ***RH*** und ***T*** zu verpacken, wobei hierbei erfindungsgemäß dies als ein Verpacken in ein Primärpackmittel zu verstehen ist. Beispielsweise kann hierbei eine Verpackung in Form von Blisterkarten zum Einsatz kommen. Hierbei dient der Blister als Primärpackmittel. Als geeignete Prozessparameter haben sich hierbei herausgestellt, wenn der Prozess bei ***RH*** = 15-35%, ***T*** = 15-30°C, bevorzugt bei ***RH*** = 18-27%, ***T*** = 16-28°C, besonders bevorzugt bei ***RH*** = 19-26%, ***T*** = 17-27°C, ganz besonders bevorzugt bei ***RH*** = 20-25%, ***T*** = 19-23°C durchgeführt wird. Vorteilhaft erweist es sich hierbei, wenn die Folien zu Herstellung der Blister beginnend vom letzten Herstellschritt der Folien, sowie bei der Lagerung, dem Transport, ggf. Zwischenlagerung bis zu ihrem Einsatz im Rahmen der Verblisterung des erfindungsgemäßen Arzneimittels kontrollierten klimatischen Bedingungen ausgesetzt sind. Bevorzugte klimatische Bedingungen sind hierbei für die Temperatur der Bereich von 5°C bis 45°C und für die relative Feuchte 10% r.F. bis 60% r.F.., besonders bevorzugt 5°C bis 40°C und 10% r.F. bis 50% r.F., darüber hinaus besonder bevorzugt 5°C bis 40°C und 15% r.F. bis 40% r.F., ganz besonders bevorzugt 10°C bis 30°C und 15% r.F. bis 40% r.F., ganz besonders herausragend bevorzugt 10°C bis 30°C und 20% r.F. bis 30% r.F. und ganz besonderer herausragender Art und Weise 15°C bis 30°C und 20% r.F. bis 30% r.F.

Eine als Blisterverpackung ausgestaltete Verpackungseinheit, die im Rahmen der Erfindung zur Verpackung von Inhalationskapseln, welche die erfindungsgemäßen Arzneimittelzubereitungen enthalten, verwendet werden kann, besteht in der Regel aus einer Deckfolie und einer Bodenfolie, wobei in der Bodenfolie mehrere Kavitäten ausgebildet sind. Die Deckfolie und die Bodenfolie können aus einer oder mehreren Schichten verschiedener oder gleicher Materialien aufgebaut sein. Die Deckfolie wird mit der Bodenfolie z. B. durch Kleben, Schweißen oder Versiegeln abgedichtet verbunden. Die Deckfolie und/oder die Trägerfolie ist in der Regel als Metall- und/oder Kunststoff- und/oder Papierfolie ausgebildet. Diese Materialien können in mehreren Schichten vorhanden sein. Typische Metallfolien umfassen beispielsweise Aluminiumfolien und Aluminiumverbundfolien, die aus Aluminium und z.B. einem Kunststoff gefertigt sind. Als Material für die Kunststofffolien kann Polyvinylchlorid (PVC) Cycloolefin-Copolymer (COC), Polychlortrifluorethylen (PCFE), Polyethylen (PE), Polypropylen (PP), Polyethylenterephtalat (PET), Polycarbonat (PC), Polyester (UP), Polyacrylat, Polyamid (PA) oder andere Kunststoffe verwendet werden. Bevorzugt ist hierbei die Verwendung der Kunststoffe PE und PP, besonders bevorzugt PP. Häufig besteht ein Blister aus einer Deckfolie aus Aluminium, die die Bodenfolie zur Aufnahme des pharmazeutischen Produktes bzw. Wirkstoffes verschließt. Diese tiefgezogene Bodenfolie kann ebenfalls eine Aluminiumfolie umfassen, um den Eintritt von Wasser in die Kavität zur Aufnahme des pharmazeutischen Produktes zu verhindern. Zur Schaffung einer weiteren Diffusionsbarriere bzw. zur Erhöhung der mechanischen Stabilität des Blisters kann optional zumindest die Aluminiumfolien der Bodenfolie ein oder beidseitig mit weiteren Kunststoff- und/oder Papierfolien bedeckt sein.

Zur Verwendung im Rahmen der vorliegenden Erfindung sind insbesondere Blisterverpackungen mit folgender Schichtenabfolge vorgesehen. Die Deckfolie ist aus Aluminium gefertigt und weist eine Dicke von 10 bis 80 Mikrometer, bevorzugt von 20 bis 50 Mikrometer, insbesondere von 30 bis 40 Mikrometer auf. Die Deckfolie ist mittels eines Heißsiegellackes mit der die Kavitäten aufweisenden Bodenfolie abgedichtet verbunden. Die Bodenfolie besteht auf der Produkt berührenden Seite aus einer PVC-, PP-, PE-Schicht o. Ä. mit einer Dicke zwischen 10 bis 200 Mikrometer, bevorzugt zwischen 15 und 50 Mikrometer, insbesondere zwischen 20 und 40 Mikrometern. Diese Folie ist mit einer Aluminiumfolie verbunden, deren Dicke bevorzugt 30 bis 60 Mikrometer, vorteilhafterweise 35 bis 50 Mikrometer beträgt. An die Aluminiumfolie schließt sich eine Polyamid-Folie an, die eine Dicke zwischen 10 und 40 Mikrometern, bevorzugt 15 bis 30 Mikrometer aufweist. Bei einer alternativen Bodenfolie ist die PCV-Folie auf der dem Produkt zugewandten Seite durch eine Polypropylenfolie oder dergleichen ersetzt. Bei einer bevorzugten Blisterverpackung besteht die Deckfolie aus einer 38 $\mu$m dicken Aluminiumfolie und dem Heißsiegellack. Die Bodenfolie ist auf der dem pharmazeutischen Produkt zugewandten Seite aus einer 30 $\mu$m dicken PVC-Folie, einer sich daran anschließenden 45 $\mu$m dicken Aluminium-Folie sowie einer außenseitigen 20 $\mu$m dicken Polyamidfolie gefertigt.

Es ist von Vorteil, wenn die Erfindungs-gemäßen Arzneimittelzubereitungen, die beispielsweise in Blisterkarten verpackt sind, mittels eines zusätzlichen Pouches zum Zwecke der Langzeitlagerung zusätzlich geschützt werden. Dieser zusätzliche Schutz und somit folglich der Pouch entspricht einem Sekundärpackmittel.

Dabei kann der Pouch z. B. als Schlauchbeutel oder 4-Randsiegelbeutel derart ausgestaltet werden, dass die Oberfolie und die Unterfolie z. B. durch Kleben, Schweißen oder Versiegeln abgedichtet verbunden wird. Die Folien sind in der Regel als Metall- oder Metall-Kunststoffverbund- oder Metall-Kunststoff-Papierverbundfolie ausgebildet. Diese Materialien können in mehreren Schichten vorhanden sein. Typische Metallfolien umfassen beispielsweise Aluminiumfolien und Aluminiumverbundfolien, die aus Aluminium und z.B. einem Kunststoff gefertigt sind. Als Material für die Kunststofffolien kann Polyvinylchlorid (PVC) Cycloolefm-Copolymer (COC), Polychlortrifluorethylen (PCFE), Polyethylen (PE), Polypropylen (PP), Polyethylenterephtalat (PET), Polycarbonat (PC), Polyester (UP), Polyacrylat, Polyamid (PA) oder andere Kunststoffe verwendet werden. Erfindungsgemäß bevorzugt sind ferner solche Folien, die eine Permeationsrate für Wasser von kleiner 5g/$m^2$d, bevorzugt kleiner 2g/$m^2$d, besonders bevorzugt kleiner 1g/$m^2$d, ganz besonders bevorzugt kleiner 0,1g/$m^2$d und besonders herausragend bevorzugt kleiner 0,05g/$m^2$d aufweisen. Hierbei versteht man

unter Permeationsrate ein Maß für die Wasserdampfpermeabilität durch die Folie nach DIN 53122 bei 38°C und 90% rel. Luftfeuchtigkeit.

Dabei ist bevorzugt, wenn der Pouch derart ausgestaltet ist, dass in demselben ein vorbefeuchtetes Trockenmittelpäckchen neben einer oder mehr als einer Blisterkarte eingelegt wird. Ein solches vorbefeuchtetes Trockenmittelpäckchen kann durch einen zusätzlichen Konditionierschritt erhalten werden.

Die Zeitdauer für diesen zusätzlichen Konditionierungsschritt wird, um ein vorbefeuchtetes Trockenmittelpäckchen zu erhalten, abhängig vom Erreichen eines Feuchtigkeitsgleichgewichts zwischen dem Trockenmittel und der umgebenden Atmosphäre bemessen. Dabei wird die Restfeuchte der Feuchtigkeitsatmosphäre entsprechend der gewünschten minimalen Restfeuchte in der Verpackungseinheit nach dem Verpacken der pharmazeutischen Wirkstoffformulierung eingestellt. Damit kann das Trockenmittel gezielt, entsprechend den optimalen Lagerbedingungen des Wirkstoffes bzw. der Wirkstoffformulierung, vorkonditioniert werden, beziehungsweise der entsprechende $a_w$-Wert eingehalten werden.

In weiterer Ausgestaltung des Konditionierschrittes zur Vorbefeuchtung der Trockenmittelpäckchen wird eine homogene Verteilung der Feuchtigkeitsatmosphäre während des zusätzlichen Konditionierungsschrittes über dem Trockenmittel eingestellt. Dadurch ist sichergestellt, dass das gesamte Trockenmittel den gleichen Restfeuchtegehalt aufweist und nach dem Verpacken keine Änderung gegenüber dem gewünschten, voreingestellten Feuchtebereich infolge von Ausgleichsprozessen auftreten kann.

Wirkungsvoll und effizient hinsichtlich einer möglichst kurzen Prozesszeit und Homogenisierung kann dies geschehen, wenn das Trockenmittel, beispielsweise in Form eines losen Granulats oder abgepackt in atmungsaktiven Beuteln, während des zusätzlichen Konditionierungsschrittes innerhalb der Feuchtigkeitsatmosphäre umgewälzt wird, was beispielsweise in Trommel- oder Rührvorrichtungen geschehen kann.

Eine Verpackungseinheit zur Aufnahme einer pharmazeutischen Wirkstoffformulierung, die zusätzlich ein Trockenmittel enthält, das mittels des zuvor beschrieben Verfahrens vorkonditioniert wurde, bietet die Möglichkeit, hinsichtlich des Feuchtebereichs besonders empfindliche Arzneimittel sicher zu lagern.

Unter einem vorbefeuchteten Trockenmittelpäckchen kann hierbei beispielsweise eine abgepackte Menge an Silicagel, Molekularsieb (Zeolithe) oder Betonit verstanden werden, welches eine Wasserbeladung aufweist. Legt man das erfindungsgemäße Trockenmittelpäckchen in ein abgeschlossenes Gefäß (z.B. Pouch), so bedingt diese Wasserbeladung, dass die relative Feuchte in diesem abgeschlossenen Gefäß bei 25°C einen Wert zwischen 10% bis 40% aufweist. Dies kann dadurch erreicht werden, dass besagtes Trockenmittelpäckchen vor dem Einsatz als Hilfskomponente der erfindungsgemäßen verpackten Arzneimittelzubereitung beispielsweise in einer Klimakammer bei 15-30°C und 15%-35% r.F., bevorzugt bei 20-28°C und 15-30% r.F., besonders bevorzugt bei 23-27°C und 20-30% r.F. vorkonditioniert wird. In Abhängigkeit der Strömungsbedingungen (Luftwechselrate) der zum Einsatz kommenden Klimakammer sowie der Substanz-spezifischen Sorptionskinetik des zum Einsatz kommenden Trockenmittels, ist ein Gleichgewicht der Wasserbeladung besagten Trockenmittels üblicherweise nach 8h, bevorzugt nach 16h, besonders bevorzugt nach 24h erreicht.

Ebenso können erfindungsgemäße Arzneimittelzubereitungen derart verpackt werden, dass gemäß obigen Prozessschritt zur Konditionierung der Bulkware (z.B. abgefüllte Inhalationskapseln), dieselben in eine HD-PE-Flasche, welche eine vorbefeuchtete Trockenpatrone enthält (Prozessparameter zur Vorbefeuchtung gemäß den Prozessparametern zur Bereitstellung der Trockenmittelpäckchen, welche in erfindungsgemäße Pouchverpackungen eingelegt werden können), verpackt und geschützt werden. Somit kann auch mit einer solchen Verpackungskonfiguration sichergestellt werden, dass die erfindungsgemäße Eigenschaft der Arzneimittelzubereitung gewährleistet ist.

In einer weiteren Alternative ist es auch möglich, erfindungsgemäße vorkonditionierte und in Blister verpackte Arzneimittelzubereitungen zur Sicherstellung eines geeigneten $a_w$-Wertes (gemessen direkt über der verpackten Arzneimittelzubereitung) in einem Pouch zu verpacken, wobei durch beispielsweise Begasung des Pouches mit konditionierter Luft die Langzeitlagerfähigkeit sichergestellt wird. Zum Zwecke der Begasung eines solchen Pouches erweist sich erfindungsgemäß konditionierte Luft als vorteilhaft, welche beispielsweise eine Wasserlast von 10%-35% r.F., bezogen auf 25°C, bevorzugt eine Wasserlast 10%-30% r.F., bezogen auf 25°C, besonders bevorzugt eine Wasserlast 10%-25% r.F., bezogen auf 25°C und ganz besonders bevorzugt eine Wasserlast 15%-25% r.F., bezogen auf 25°C aufweist. Hierbei sind technisch absolute Wasserlasten in der konditionierten Luft zur Begasung des Pouches erfinderisch ebenso einsetzbar, wie sie sich durch Umrechnung gemäß Mollier-Diagramm aus dem Stand der Technik ergeben.

Es werden ausdrücklich Verpackungskonfigurationen, welche sicherstellen, dass die erfindungsgemäßen Arzneimittelzubereitungen während der Laufzeit des Medikamentes bis einschließlich der Entnahme des Arzneimittels aus dem Packmittel, bevorzugt während eines Zeitraumes von mindestens 18 Monaten Lagerung einen $a_w$-Wert zwischen 0.05 und 0.5, bevorzugt zwischen 0.10 und 0.45, besonders bevorzugt zwischen 0.15 und 0.40 und Ganz besonders bevorzugt zwischen 0.15 und 0.35 aufweisen, hiermit ebenso umfasst. Dabei erfüllen erfmdungsgemäße Arzneimittelzubereitungen die Stabilitätsanforderungen bei 25°C/18 Monaten der ICH-Guideline Q1A (R2), Februar 2003.

Ferner betrifft die vorliegende Erfindung ein Inhalationskit, bestehend aus einer oder mehrere der vorstehend beschriebenen, durch einen Gehalt an erfindungsgemäßem Inhalationspulver gekennzeichneten Kapseln in Verbindung

mit dem Inhalator gemäß Figur 2.

Die vorliegende Erfindung betrifft ferner die Verwendung der vorstehend genannten, durch einen Gehalt an erfindungsgemäßem Inhalationspulver gekennzeichneten Kapseln, zur Herstellung eines Arzneimittels zur Behandlung von Atemwegserkrankungen, insbesondere zur Behandlung von COPD und/oder Asthma.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma, dadurch gekennzeichnet, dass der in Figur 2 dargestellte Inhalator zur Anwendung gelangt.

**Ausgangsmaterialien**

**I) Hilfsstoff:**

**Ia. :**

In den nachfolgenden Beispielen 1 bis 5 wird als Hilfsstoff Lactose-Monohydrat verwendet. Dieser kann beispielsweise von der Firma Borculo Domo Ingredients, Borculo/NL unter der Produktbezeichnung *Lactochem Extra Fine Powder* bezogen werden. Die erfindungsgemäßen Spezifikationen für die Teilchengröße und die spezifische Oberfläche werden von dieser Lactosequalität erfüllt. Ferner weist diese Lactose die vorstehend genannten, für Lactose erfindungsgemäß bevorzugten Lösungsenthalpie-Werte auf Beispielsweise wurden in den nachfolgenden Beispielen Lactosechargen verwendet, die die folgenden Spezifikationen aufwiesen:

a) mittlere Teilchengröße: 17,9 μm; 10 %-Feinanteil: 2,3 μm; spezifische Oberfläche:

0,61 $m^2$/g; oder

b) mittlere Teilchengröße: 18,5 μm; 10 %-Feinanteil: 2,2 μm; spezifische Oberfläche:

0,83 $m^2$/g;

c) mittlere Teilchengröße: 21,6 μm; 10 %-Feinanteil: 2,5 μm; spezifische Oberfläche:

0,59 $m^2$/g;

d) mittlere Teilchengröße: 16,0 μm; 10 %-Feinanteil: 2,0 μm; spezifische Oberfläche:

0,79 $m^2$/g

**Ib. :**

In den nachfolgenden Beispielen 6 bis 9 wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden. Diese Lactose ist gekennzeichnet durch eine mittlere Teilchengröße von etwa 30 bis 35 μm. Verwendete Lactosechargen 200M wiesen beispielsweise eine mittlere Teilchengröße von 31 μm bei einem 10%-Feinanteil von 3,2μm oder auch eine mittlere Teilchengröße von 34μm bei einem 10%-Feinanteil von 3,5μm auf.

In den nachfolgenden Beispielen 6 bis 9 wird als feinerer Hilfsstoff Lactose-Monohydrat mit einer mittleren Teilchengöße von 3-4μm verwendet. Dieses kann durch gängige Verfahren (Mikronisieren) aus kommerziell erhältlichem Lactose-Monohydrat, beispielsweise der vorstehend genannten Lactose 200M erhalten werden. Verwendete mikronisierte Lactosechargen wiesen beispielsweise eine mittlere Teilchengröße von 3,7μm bei einem 10%-Feinanteil von 1,1μm oder auch eine mittlere Teilchengröße von 3,2μm bei einem 10%-Feinanteil von 1,0μm auf.

**Ic. :**

In den nachfolgenden Beispielen 10 bis 12 wird als Hilfsstoff Lactose-Monohydrat (200M) verwendet. Dieser kann beispielsweise von der Firma DMV International, 5460 Veghel/NL unter der Produktbezeichnung Pharmatose 200M bezogen werden. Diese Lactose ist gekennzeichnet durch eine mittlere Teilchengröße von etwa 30 bis 35 μm.

**Id. :**

In den nachfolgenden Beispielen 13 bis 19 wird als Hilfsstoff Lactose-Monohydrat (200M) verwendet. Dieser kann beispielsweise von der Firma DMV International, 5460 Veghel/NL unter der Produktbezeichnung Respitose ML003 bezogen werden. Diese Lactose ist gekennzeichnet durch eine mittlere Teilchengröße von etwa 30 bis 35 μm. Verwendete Lactosechargen Respitose ML003 wiesen beispielsweise eine mittlere Teilchengröße von 31μm bei einem 10%-Feinanteil von 3,2μm oder auch eine mittlere Teilchengröße von 34μm bei einem 10%-Feinanteil von 3,5μm auf.

**II) Darstellung von erfindungsgemäßem Salmeterolzinafoat:**

20 g Salmeterol-Base und 9,1 g 1-Hydroxy-2-Naphthoesäure werden in 260 ml Ethanol abs. und 260 ml tert. Butylmethylether suspendiert. Die Suspension wird auf 55-56°C erwärmt und gerührt, bis eine klare Lösung entstanden ist. Die Lösung wird filtriert und der Filter mit 30 ml Ethanol abs. und 30 ml tert. Butylmethylether ausgespült. Das Filtrat wird auf 38°C abgekühlt und mit einigen Kristallen Salmeterolxinafoat angeimpft. Die Lösung wird 1 h bei 34-37°C gerührt, wobei die Kristallisation einsetzt. Die Suspension wird auf 1-3°C abgekühlt und ca. 30 min bei dieser Temperatur gerührt. Der Niederschlag wird über einen Nutschfilter abgesaugt und mit 20 ml Ethanol und 120 ml tert. Butylmethylether gewaschen. Der Feststoff wird bei 45°C im Stickstoffstrom getrocknet. Ausbeute: 26 g (89,5%)
Das so erhaltene kristalline Salmeterolxinafoat weist ein Stampfvolumen von 0,27 g/cm$^3$ auf.

**III) Mikronisierung von Salmeterolxinafoat:**

**IIIa)**

Das gemäß der vorstehenden Vorgehensweise erhältliche Salmeterolxinafoat wird mit einer Luftstrahlmühle vom Typ MC JETMILL 50 Firma Jetpharma; Via Sotto Bisio 42 a/c, 6828-Balerna, Schweiz, mikronisiert. Unter Verwendung von Stickstoff als Mahlgas werden dabei beispielsweise die folgenden Mahlparameter eingestellt:

Mahldruck 7,5 bar, Speisedruck 8,0 bar.
Zufuhr (des kristallinen Salmeterolxinafoats) bzw. Fließgeschwindigkeit) 40 g/min.

**IIIb)**

Das gemäß der vorstehenden Vorgehensweise erhältliche Salmeterolxinafoat wird mit einer Luftstrahlmühle vom Typ Chrispro Jet-Mill MC200, Mahlkammergröße = 200 mm mit Mahldüsen 2,25 mm, Firma Micro Macinazione SA, Via Cantonale, 6995 Molinazzo di Monteggio (CH). Unter Verwendung dieses Geräts wird der Mahlprozess mit folgenden Mahlparametem durchgeführt:

Mahldruck: 4 bar; Speisedruck: 4.5 bar: Zufuhr des Mahlguts: etwa 200 g/min

Das so erhaltene mikronisierte Salmeterolxinafoat weist ein Stampfvolumen von 0,19 g/cm$^3$ auf.

**IV) Mikronisierung von kristallinem Tiotropiumbromid-monohydrat:**

Das gemäß der WO 02/30928 erhältliche kristalline Tiotropiumbromid-monohydrat wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:

Mahldruck: 5,5 bar; Speisedruck: 5,5 bar;
Zufuhr (des kristallinen Monohydrats) bzw. Fließgeschwindigkeit: 19 g/min.

Das erhaltene Mahlgut wird anschließend auf Hordenblechen (z.B. Hordentrocknerbleche Firma Glatt, 79589 Binzen, Deutschland) in einer Schichtdicke von etwa 1 cm ausgebreitet und für 24 - 24,5 Stunden den folgenden Klimabedingungen unterworfen:

Temperatur: 25 - 30 °C; Relative Feuchte: 70-80%.

**Meßmethoden:**

**I) Röntgenstrukturanalyse von Salmeterolxinafoat:**

Messgerät und Einstellungen:

Das Röntgenpulverdiagramm wurde im Rahmen der vorliegenden Erfindung aufgenommen mittels eines BRUKER D8 ADVACED - Diffraktometers, ausgerüstet mit einem ortsempfindlichen Detektor (=OED) und einer Cu-Anode als Röntgenquelle ($CuK_\alpha$ - Strahlung, $\lambda$ = 1.5418 Å, 40 kV, 40 mA).

Das für das erfindungsgemäße Salmeterolxinafoat erhaltene Röntgenpulverdiagramm ist in Figur 1 dargestellt. Nachstehende Tabelle 1 fasst die bei dieser spektroskopischen Analyse erhaltenen Daten zusammen:

Tabelle 1: Intensitäten (normalisiert) der Röntgenreflexe

| 2 Θ [°] | d [Å] | $I/I_o$ [%] |
|---|---|---|
| 4,10 | 21,5 | 100 |
| 8,27 | 10,7 | 4 |
| 10,51 | 8,41 | 12 |
| 10,86 | 8,14 | 6 |
| 11,71 | 7,55 | 3 |
| 12,68 | 6,98 | 5 |
| 12,98 | 6,82 | 5 |
| 13,54 | 6,54 | 4 |
| 13,81 | 6,41 | 5 |
| 14,19 | 6,23 | 3 |
| 14,69 | 6,03 | 2 |
| 15,59 | 5,68 | 3 |
| 17,23 | 5,14 | 18 |
| 17,73 | 5,00 | 3 |
| 18,69 | 4,74 | 9 |
| 19,47 | 4,56 | 4 |
| 20,40 | 4,35 | 11 |
| 21,24 | 4,18 | 4 |
| 22,14 | 4,01 | 16 |
| 23,24 | 3,82 | 3 |
| 23,77 | 3,74 | 4 |
| 24,50 | 3,63 | 22 |
| 25,93 | 3,43 | 4 |
| 26,23 | 3,40 | 3 |
| 27,34 | 3,26 | 3 |
| 28,26 | 3,16 | 3 |
| 28,70 | 3,11 | 3 |
| 29,80 | 3,00 | 3 |
| 31,21 | 2,86 | 4 |
| 33,08 | 2,71 | 3 |

(fortgesetzt)

| 2 Θ [°] | d [Å] | $I/I_o$ [%] |
|---|---|---|
| 35,76 | 2,51 | 3 |

In vorstehender Tabelle steht der Wert "2 Θ [°]" für den Beugungswinkel in Grad und der Wert "d [Å]" für die bestimmten Gitterebenenabstände in Å.

**II) Partikeigrößenbestimmung von Tiotropium Monohydrat, mikronisiert und Salmeterolxinafoat, mikronisiert:**

Messgerät und Einstellungen:

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec (Partikelgrößenbestimmung mittels Fraunhoferbeugung) |
| Dispergiereinheit: | Trockendispergierer RODOS mit Saugtrichter, Sympatec |
| Probenmenge: | 200 mg ± 150 mg |
| Produktzufuhr: | Schwingrinne Vibri, Fa. Sympatec |
| Frequenz d. Vibrationsrinne: | bis 100 % ansteigend |
| Dauer der Probenzufuhr: | 15 bis 25 sek. (im Fall von 200 mg) |
| Brennweite: | 100 mm (Messbereich: 0,9-175 μm) |
| Messzeit/Wartezeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne so variiert, dass die Zufuhr der Probe möglichst kontinuierlich erfolgt. Die Produktmenge darf aber auch nicht zu groß sein damit eine ausreichende Dispergierung erreicht wird.

**III) Partikelgrößenbestimmung von Laktose:**

Messgerät und Einstellungen

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec (Partikelgrößenbestimmung mittels Fraunhoferbeugung) |
| Dispergiereinheit: | Trockendispergierer RODOS mit Saugtrichter, Sympatec |
| Probenmenge: | 200 mg ± 100 mg |
| Produktzufuhr: | Vibrationsrinne Typ VIBRI, Sympatec |
| Frequenz d. Vibrationsrinne: | 100 % ansteigend |
| Brennweite: | 200 mm (Messbereich: 1,8 - 350 μm) |

(fortgesetzt)

| | |
|---|---|
| Messzeit/Wartezeit: | ca. 10 s (im Falle von 200 mg) |
| Zykluszeit: | 10 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen. Mit einem weiteren Kartenblatt werden alle größeren Agglomerate zerstoßen. Das Pulver wird in die Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne möglichst kontinuierlich auf 100 % gegen Ende der Messung gesteigert.

**IV) Bestimmung der spezifischen Oberfläche von Tiotropiumbromid-Monohydrat, mikronisiert und Salmeterolxinafoat, mikronisiert (Mehr-Punkt-BET-Methode):**

Messgerät:

TriStar 3000, Firma Micromeretics

Probenvorbereitung:

- Verwenden von Probenröhrchen 1/2 inch
- Einwaage: ca. 1 - 3 g (so groß wie möglich → rote Markierung nicht überschreiten)
- Ausheizen der Probe für mind. 4 h bei 40 °C
  Vorbereitungsstation: VacPrep 061

| | |
|---|---|
| | Methode: multi-8 point p/p 0:0,05 - 0,12 |
| • Messgas: | Stickstoff |
| • Totvolumen: | zu bestimmen mittels Helium |
| • Sättigungsdruck p0: | zu bestimmen |
| | Messinterval: 120 Min |
| | Temperatur: Siedetemperatur Stickstoff bei Umgebungsdruck (~77 K) |
| • Evakuierungsrate: | 50,0 mmHg/sec |
| • Evakuierungszeit: | 0,5 h |
| • Gleichgewichtsintervall: | 15 sec |

Durchführung:

Es werden 2 Substanzproben vermessen.

Die Messwerte werden einzeln aufgelistet und anschließend gemittelt.

**V) Bestimmung der Lösungswärme der Laktose (Lösungsenthalpie) $E_c$:**

Die Bestimmung der Lösungsenthalpie erfolgt mittels eines Lösungskalorimeter *2225 Precision Solution Calorimeter* der Fa. Thermometric.

Die Lösungswärme wird anhand der - infolge des Löseprozesses - auftretenden Temperaturänderung und der aus der Basislinie berechneten systembedingten Temperaturänderung berechnet. Vor und nach dem Ampullenbruch wird jeweils eine elektrische Kalibrierung mit einem integrierten Heizwiderstand genau bekannter Leistung durchgeführt.

Hierbei wird eine bekannte Wärmeleistung über einen festgelegten Zeitraum an das System abgegeben und der Temperatursprung ermittelt.

Messgerät: und Einstellungen

| | |
|---|---|
| Lösungskalorimeter: | 2225 Precision Solution Calorimeter, Fa. Thermometric |
| Reaktionszelle: | 100 ml |
| Thermistorwiderstand: | 30,0 kΩ (bei 25 °C) |
| Rührergeschwindigkeit: | 500 U/min |
| Thermostat: | Thermostat des 2277 Thermal Activity Monitor TAM, Fa. Thermometric |
| Temperatur: | 25 °C ± 0.0001 °C (über 24h) |
| Meßampullen: | Crushing ampoules 1 ml, Fa. Thermometric |
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mK (± 10mK) temperature-offset |
| Interface: | 2280-002 TAM accessory interface 50 Hz, Fa. Thermometric |
| Software: | SolCal V 1.1 für WINDOWS |
| Auswertung: | Automatische Auswertung mit Menüpunkt CALCULATION/ ANALYSE EXPERIMENT. (Dynamik der Basislinie; Kalibrierung nach dem Ampullenbruch). |

Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5 J |
| Heizleistung: | 500 mW |
| Heizdauer: | 10 s |
| Dauer der Basislinien: | 5 min (vor und nach Heizen) |

**VI) Bestimmung des $a_w$-Wertes**:

Prinzipielles:

Zur Bestimmung des $a_w$-Werts wird die Luftfeuchte nach Erreichen des Feuchtegleichgewichts bezogen auf 25°C unmittelbar über einer Probe (Wasserdampf-Partial-Differenzdruck) gemessen. Diese verhält sich proportional zum $a_w$-Wert. Eine aussagekräftige $a_w$-Wertmessung ist nur möglich, wenn die Probe während der Messung eine konstante Temperatur aufweist.

Die Bestimmung der Luftfeuchte, mit Hilfe dessen der $a_w$-Wert berechnet werden kann, muss dabei der Art erfolgen, dass mit ausreichender Genauigkeit auch kleine

Probenvolumina, wie sie beispielsweise im Falle von Blisterkavitäten vorliegen, analysiert werden können. Dem Fachmann sind spezifische Verfahren diesbezüglich bekannt. Im speziellen soll im Rahmen dieser Erfindung auf das beschriebene Analyseverfahren von *Xu et al.* (Xu, H., Templeton, A.C., Zwierzynski, M., Mahajn R., Reed, R.A.; "Rapid, simultaneous determination of headspace oxygen and moisture in pharmaceutical packages using μGC", Journal of Pharmaceutical and Biomedical Anaysis, 38 (2005), 225-231) verwiesen werden bzw. auf die Methode, welche in Anlehnung an Xu et al. im folgenden aufgeführt wird. Die im Rahmen vorliegender Erfindung angegebenen Messwerte beziehen sich auf das nachfolgende Analyseverfahren, mit Hilfe dessen der erfindungsgemäß definierte Kennwert bestimmt werden kann. Alternativmöglichkeiten, die diese Aufgabe in vergleichbarer Art und Weise lösen, können prinzipiell ebenfalls zum Einsatz kommen.

Geräte / Material:

1.) Analysengerät

- Gaschromatographie-MikroGC, Modell 3000A, Hersteller / Firma Agilent
- Injektionsnadel mit seitlichem Loch (Lieferant: Teuner Analysentechnik GmbH)
- Trägergas: Helium 4.6, z.B. Fa. AirLiquide

2.) Kalibrierequipment

- Kalibrierkammern für 2 Sensoren, Hersteller / Firma Rotronic
- zertifizierte Feuchtenormale zu 55%, 10%, 20%, 35%, 50%, 65%, 75,3%, 80%, Hersteller / Firma Rotronic

3.) Klimaüberwachung

- Klimaschrank KBF240, Hersteller / Firma Binder
- Klimamessgerät, z.B. Modell HygroPalm2 mit Sensor Modell HygroClip
- digitales Thermometer, z.B. Modell DigiTherm

4.) Material zur Probenvorbereitung

- Abdichtung des Einstichloches: Powerstrip, Hersteller / Firma Tesa
- Tesafilm, Hersteller / Firma Tesa
- Dorn bzw. Körner

Allgemeines:

Die Messungen werden bei konstanten Bedingungen von 25°C/50% r.F. in einem Klimaschrank durchgeführt.

Gaschromatographische Betriebsbedingungen der MikroGC. Modell 3000A, Fa. Agilent:

Die mit 2 Modulen versehene mikroGC wird seitens Modul (Kanal) A - Kanal zur Analyse von Stickstoff und Sauerstoff - deaktiviert und mittels Modul (Kanal) B - Kanal zur Analyse von Wasser - betrieben. Es ist zu berücksichtigen, dass diverse Einstellungen, wie z.B. Ladezeit und Probeneinlasstemperatur jedoch für das Gesamtsystem in Modul A eingetragen werden müssen. Es sei zusätzlich auf die Angaben des Geräteherstellers verwiesen.

Das Trägergas wird mit einem Druck von 5,5 bar zur Verfügung gestellt.

Tabelle 2: Tabellarische Zusammenstellung der gaschromatographischen Bedingungen:

| Modul/Kanal/Säule | | A | B |
|---|---|---|---|
| Säulentyp | | Molekularsieb | Stabilwax |
| Säulendimension | | 10m x 320µm x 12µm | 10m x 250µm x 0,5µm |
| Trennung von | | [$O_2$ und $N_2$] | [$H_2O$] |
| Probeneinlasstemperatur | | 90 | - |
| Inj ektortemperatur | | 100 | 100 |
| Säulentemperatur | | 100 | 100 |
| Ladezeit | | 1 | - |
| Injektionszeit | | 20 | 200 |
| Laufzeit | | 90 | 90 |
| Nachanalysezeit | | 5 | 5 |
| Druckequilibrierzeit | | 0 | 0 |

(fortgesetzt)

| Modul/Kanal/Säule | | A | B |
|---|---|---|---|
| Säulendruck | | 2,0 | 1,0 |
| Nachanalysedruck | | 2,0 | 1,0 |
| Detektorglühdraht | | deaktiviert | aktiviert |
| Detektorempfindlichkeit | | Standard | Standard |
| Detektordatenrate | | 100 | 100 |
| Basislinienverschiebung | | 0 | 0 |
| Rückspülzeit | | 0 | n/a |
| Dauer Chromatographie | | 1,5 | 1,5 |
| Retentionszeit Wasser | | - | 0,90 |
| Injektortyp | | Rückspülung | Zeit |
| Trägergas | | Helium 4.6 | Helium 4.6 |
| Detektortyp | | WLD | WLD |
| Einlasstyp | | beheizt | beheizt |

Kalibrierung zur Ermittlung einer Kalibriergeraden:

Arbeitstäglich sind frisch angesetzte Kalibrierlösungen zu verwenden. Die Herstellung der Kalibrierproben (unterschiedliche relative Feuchte Konzentrationen) erfolgt gemäß Kalibrieranleitung der Fa. Rotronic AG. Dafür müssen Feuchtenormal und Kalibrierkammer auf die gleiche Temperatur wie das mikroGC gebracht werden. Der Inhalt einer Ampulle ist in die Mitte des Textilteils im Deckel der Kalibrierkammer zu entleeren und die Kalibrierkammer ist zu schließen. Nach der Equilibrierzeit werden mit der Injektionsnadel der mikroGC durch eine mit Tesafilm oder vergleichbarem Material abgedichteten Stelle gemäß den oben beschriebenen Gaschromatographischen Bedingungen mindestens 7 Injektionen entnommen. Die ersten beiden Injektionen werden verworfen, die restlichen sind zur Auswertung der Erstellung der Kalibriergeraden heranzuziehen.

Messung der Probe zur Bestimmung des $a_W$-Wertes:

Die Einstichstelle am Packmittel (z.B. Pouch oder Blisternapf) wird mit einem "Powerstrip", Fa. Tesa versehen, auf welchem ein Tesafilm geklebt wird. PE-Flaschen sind gegebenenfalls durch den Powerstrip und Tesafilm mit einem Körner oder ähnlichem Werkzeug zu lochen, wobei unmittelbar nach diesem Lochen die Einstichstelle mit einem Tesafilm zu verschließen ist.

Die eigentliche Messung erfolgt durch Einstechen der Injektionsnadel der mikroGC direkt im Anschluss an die Vorbereitung der Einstichstellen gemäß den Gaschromatographischen Bedingungen (siehe auch Tabelle 2). In Abhängigkeit des freien Luftvolumens der zu vermessenden Probe werden 3 bis 5 Injektionen entnommen unter Beachtung der Temperatur während der Messung. Hierbei ist für die weitere Auswertung jeweils die erste Injektion (bei 3 Injektionen) bzw. die ersten beiden Injektionen (bei mehr als 3 Injektionen) pro zu vermessendem Muster zu verwerfen.

Auswertung / Berechnung des $a_W$-Wertes:

Die Auswertung erfolgt gegen die Kalibriergerade. Dabei ergibt sich der $a_W$-Wert als der gemittelte Zahlenwert der abgelesenen relativen Feuchte geteilt durch 100 bezogen auf die Messtemperatur (hier 25°C).

**Darstellung der erfindungsgemäßen Pulverformulierungen:**

**I) Apparatives**

Zur Herstellung der Inhalationspulver können beispielsweise die folgenden Maschinen und Geräte Verwendung finden:

Mischbehälter bzw. Pulvermischer:

- Turbulamischer 2 L, Typ 2C; Hersteller Willy A. Bachofen AG, CH-4500 Basel

*Alternative Mischer*:

- Low Shearmischer (Schrauben-Mischer, Ruberg-Mischer)
- High Shearmischer (Internsiv-Mischer, Diosna-Mischer)

Handsieb:

- 0,135 mm Maschenweite

*Alternative Siebgeräte:*

- Siebgranulatoren z.B. Quadro Comil oder Glatt Schnellsieb

Die Befüllung der leeren Inhalationskapseln mittels Wirkstoffkombination-haltigem Inhalationspulver kann händisch oder maschinell erfolgen. Beispielsweise kann nachstehendes Gerät verwendet werden.

Kapselfüllmaschine:

MG2, Typ G100, Hersteller: MG2 S.r.l, 1-40065 Pian di Macina di Pianoro (BO), Italien

Verfahren zur Einstellung eines spezifischen Eigen-Wassergehaltes alternativen):

- Konditionierung der Kapseln auf Horden (z.B. Hordentrocknerbleche Firma Glatt, 79589 Binzen, Deutschland) im Klimaraum
- Konditionierung der Kapseln in Klimakammern z.B. Weiss Klimakammer
- Konditionierung der Kapseln in Durchlauftrocknern z.B. O(n)L(ine)D(ryer) BI
- Konditionierung der Kapseln in Trocknungs-Containem durch konditionierte Luft
- Konditionierung der Kapseln in Trocknungs-Containern durch geeignete vorkonditionierte Trocknungsmittel (z.B. vom Typ Zeolith, Silikagel, Bentonit etc.

oder vom Typ ungesättigte Salze / gesättigte Salz-/Salzlösungssysteme) Konditionierung der Kapseln in dicht schließenden Beuteln (z.B. Alu-Beutel, beinhaltend geeignete vorkonditionierte Trocknungsmittel (z.B. vom Typ Zeolith, Silikagel, Bentonit etc. oder vom Typ ungesättigte Salze / gesättigte Salz-/Salzlösungssysteme mit Zusätzen vorgelegt in Einhüllungen, die einen Flüssigkeitsaustritt verhindern)

**II) Experimentelle Beispiele**

**Beispiel 1:**

Pulvermischung :

Zur Herstellung der Pulvermischung werden 298,63 g Hilfsstoff, 0,28 g mikronisiertes Tiotropiumbromid-monohydrat und 1,09 g mikronisiertes Salmeterolxinafoat eingesetzt. In den daraus erhaltenen 300 g Inhalationspulver betragen die Wirkstoffanteile 0,09 % **1** und 0.36 % **2**.

Über ein Handsieb mit einer Maschenweite von 0,315 mm werden in einen geeigneten Mischbehälter ca. 40-45 g Hilfsstoff vorgelegt. Anschließend werden abwechselnd Tiotropiumbromid-monohydrat 1 in Portionen von ca. 40-70 mg und Hilfsstoff in Portionen von etwa 40-45 g schichtweise eingesiebt. Die Zugabe des Hilfsstoffs und des Wirkstoffs 1 erfolgt in 4 bis. 7 Schichten.
Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Anschließend werden über ein Handsieb mit einer Maschenweite von 0,315 mm in einen geeigneten Mischbehälter ca. 40-45 g der nach obiger Vorgehensweise erhältlichen, den Wirkstoff 1 enthaltenden Pulvermischung vorgelegt. Danach werden abwechselnd Salmeterolxinafoat 2 in Portionen von ca. 170-250 mg und den Wirkstoff 1 enthaltende Pulvermischung in Portionen von etwa 40-45 g schichtweise eingesiebt. Die Zugabe der den Wirkstoff 1 enthaltenden Pulvermischung und des Wirkstoffs 2 erfolgt in 4 bis 7 Schichten.
Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß oder in Analogie zu der in Beispiel 1 beschriebenen Vorgehensweise können solche Inhalationspulver erhalten werden, die nach Befüllung der entsprechenden Kunststoffkapseln beispielsweise zu den nachstehenden Inhalationskapseln fuhren:

**Beispiel 2:**

| | |
|---|---|
| Tiotropiumbromid-monohydrat: | 0,00937 mg |
| Salmeterolxinafoat | 0,03632 mg |
| Lactose Monohydrat: | 5,45431 mg |
| Polyethylen-Kapseln: | 100.0 mg |
| Total: | 105,5 mg |

**Beispiel 3:**

| | |
|---|---|
| Tiotropiumbromid-monohydrat: | 0,004685 mg |
| Salmeterolxinafoat | 0,03632 mg |
| Lactose Monohydrat: | 5,958995 mg |
| Polyethylen-Kapseln: | 100 mg |
| Total: | 106 mg |

**Beispiel 4:**

| | |
|---|---|
| Tiotropiumbromid-monohydrat: | 0,00625 mg |
| Salmeterolxinafoat | 0,03632 mg |
| Lactose Monohydrat: | 9,95743 mg |
| Polyethylen-Kapseln: | 100 mg |
| Total: | 110 mg |

**Beispiel 5:**

| | |
|---|---|
| Tiotropiumbromid-monohydrat: | 0,0125 mg |
| Salmeterolxinafoat | 0,07264 mg |
| Lactose Monohydrat: | 9,91486 mg |

(fortgesetzt)

| | |
|---|---|
| Polyethylen-Kapseln: | 100 mg |
| Total: | 110 mg |

**Beispiel 6:**

Pulvermischung (Zusammensetzung):

Zur Herstellung der Pulvermischung werden 298,63 g Hilfsstoff (95% Laktose Monohydrat "Pharmatose 200M", Hersteller DMV + 5% mikronisierte Laktose Monohydrat), 0,28 g mikronisiertes Tiotropiumbromid-monohydrat und 1,09 g mikronisiertes Salmeterolxinafoat eingesetzt. In den daraus erhaltenen 300 g Inhalationspulver beträgt der Wirkstoffanteil 0,09% **1** und 0.36 % **2**.

Hilfsstoffmischung:

Als gröbere Hilfsstoffkomponente werden 283,7g Pharmatose 200M eingesetzt. Als feinere Hilfsstoffkomponente werden 14,93g mikronisierte Lactose Monohydrat eingesetzt. In den daraus erhaltenen 298,63g Hilfsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 7.5 g bis 11,3 g Pharmatose 200M vorgelegt. Anschließend werden abwechselnd mikronisierte Laktose Monohydrat in Portionen von ca. 2 bis 5 g und Pharmatose 200M in Portionen von 2 bis 5 g schichtweise eingesiebt. Pharmatose 200M und mikronisierte Laktose Monohydrat werden in 31 bzw. in 30 Schichten (Toleranz: ±6 Schichten) zugegeben.

Die eingesiebten Bestandteile werden anschließend gemischt (Mischen: 900 Umdrehungen)

Endmischung:

Über ein Handsieb mit einer Maschenweite von 0,315 mm werden in einen geeigneten Mischbehälter ca. 40-45 g Hilfsstoffmischung vorgelegt. Anschließend werden abwechselnd Salmeterolxinafoat **2** in Portionen von ca. 90-110 mg und Hilfsstoff in Portionen von etwa 40-45 g sowie Tiotropium 1 in Portionen von ca. 22-28 mg schichtweise eingesiebt. Die Zugabe der Hilfsstoffsmischung und der Wirkstoffe **2** und **1** erfolgt in 8 bis 11 Schichten.

Die eingesiebten Bestandteile werden anschließend gemischt (Mischen: 900 Umdrehungen). Die Endmischung wird noch zweimal über ein Handsieb gegeben und jeweils anschließend gemischt (Mischen: 900 Umdrehungen).

Gemäß oder in Analogie zu der in Beispiel 6 beschriebenen Vorgehensweise können solche Inhalationspulver erhalten werden, die nach Befüllung der entsprechenden Kunststoffkapseln beispielsweise zu den nachstehenden Inhalationskapseln führen:

**Beispiel 7:**

| | |
|---|---|
| Salmeterolxinafoat | 0,03632 mg |
| Tiotropiumbromid-monohydrat: | 0,0125 mg |
| Lactose Monohydrat-Mischung: | 5,46368 mg |
| Polyethylen-Kapseln: | 100 mg |
| Total: | 105,5 mg |

**Beispiel 8:**

| | |
|---|---|
| Salmeterolxinafoat | 0,07264 mg |
| Tiotropiumbromid-monohydrat: | 0,00625 mg |
| Lactose Monohydrat-Mischung: | 9,92736 mg |

(fortgesetzt)

| | |
|---|---|
| Polyethylen-Kapseln: | 100 mg |
| Total: | 110 mg |

**Beispiel 9:**

| | |
|---|---|
| Salmeterolxinafoat | 0,07264 mg |
| Tiotropiumbromid-monohydrat: | 0,004685mg |
| Lactose Monohydrat-Mischung: | 19,92736 mg |
| Polyethylen-Kapseln: | 100 mg |
| Total: | 120,0 mg |

**Beispiel 10:**

Pulvermischung :

Zur Herstellung der Pulvermischung werden 298,63 g Hilfsstoff (Laktose Monohydrat "Pharmatose 200M", Hersteller DMV) und 0,28 g mikronisiertes Tiotropiumbromid-monohydrat und 1,09 g mikronisiertes Salmeterolxinafoat eingesetzt. In den daraus erhaltenen 300 g Inhalationspulver betragen die Wirkstoffanteile 0,09 % **1** und 0.36 % **2**.

Über ein Handsieb mit einer Maschenweite von 0,315 mm werden in einen geeigneten Mischbehälter ca. 40-45 g Hilfsstoff vorgelegt. Anschließend werden abwechselnd Tiotropiumbromid-monohydrat **1** in Portionen von ca. 40-70 mg und Hilfsstoff in Portionen von etwa 40-45 g schichtweise eingesiebt. Die Zugabe des Hilfsstoffs und des Wirkstoffs **1** erfolgt in 4 bis. 7 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Anschließend werden über ein Handsieb mit einer Maschenweite von 0,315 mm in einen geeigneten Mischbehälter ca. 40-45 g der nach obiger Vorgehensweise erhältlichen, den Wirkstoff **1** enthaltenden Pulvermischung vorgelegt. Danach werden abwechselnd Salmeterolxinafoat **2** in Portionen von ca. 170-250 mg und den Wirkstoff **1** enthaltende Pulvermischung in Portionen von etwa 40-45 g schichtweise eingesiebt. Die Zugabe der den Wirkstoff **1** enthaltenden Pulvermischung und des Wirkstoffs **2** erfolgt in 4 bis 7 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß oder in Analogie zu der in Beispiel **1** beschriebenen Vorgehensweise können solche Inhalationspulver erhalten werden, die nach Befüllung der entsprechenden Kunststoffkapseln beispielsweise zu den nachstehenden Inhalationskapseln führen:

**Beispiel 11:**

| | |
|---|---|
| Tiotropiumbromid-monohydrat: | 0,00937 mg |
| Salmeterolxinafoat | 0,03632 mg |
| Lactose Monohydrat: | 9,95431 mg |
| Polyethylen-Kapseln: | 100 mg |
| Total: | 110,0 mg |

**Beispiel 12:**

| | |
|---|---|
| Tiotropiumbromid-monohydrat: | 0,00625 mg |
| Salmeterolxinafoat | 0,03632 mg |

(fortgesetzt)

| | |
|---|---|
| Lactose Monohydrat: | 9,95743 mg |
| Polyethylen-Kapseln: | 100 mg |
| Total: | 110 mg |

**Beispiel 13:**

Pulvermischung :

Zur Herstellung der Pulvermischung werden 5725,86g Hilfsstoff, 56,22 g mikronisiertes Tiotropiumbromid-monohydrat und 217,92 g mikronisiertes Salmeterolxinafoat eingesetzt. In den daraus erhaltenen 6 kg Inhalationspulver betragen die Wirkstoffanteile 0,0937 % **1** und 0.363 % **2**.

Mittels eines Siebgranulators der Fa. Glatt, GS180 mit einer Maschenweite von 0,45 mm (Rotorgeschwindigkeit 900 U/min) werden in einen geeigneten Mischbehälter ca. 50% des Hilfsstoffs Laktose Monohydrat der Qualität Respitose ML003 (200M), DMV, vorgelegt. Anschließend werden abwechselnd Tiotropiumbromid-monohydrat **1** in einer Portion darauf folgend ca. 500g des Hilfsstoffs und dann die Gesamtmenge an **2** gefolgt von ca. 500g des Hilfsstoffesschichtweise eingesiebt. Danach folgt die Zugabe der Restmenge des Hilfsstoffs durch den Siebgranulator in den Mischbehälter. Die eingesiebten Bestandteile werden anschließend 8 min gemischt (Rührergeschwindindigkeit 90 U/min, Zerhackergeschwindigkeit 1500 U/min).

Gemäß oder in Analogie zu der in Beispiel 13 beschriebenen Vorgehensweise können solche Inhalationspulver erhalten werden, die nach Befüllung der entsprechenden Kunststoffkapseln beispielsweise zu den nachstehenden Inhalationskapseln führen:

**Beispiel 14:**

| | |
|---|---|
| Tiotropiumbromid-monohydrat: | 0,00937 mg |
| Salmeterolxinafoat | 0,03632 mg |
| Lactose Monohydrat: | 9,95431 mg |
| Polyethylen-Karseln: | 100 mg |
| Total: | 110,0 mg |

**Beispiel 15:**

| | |
|---|---|
| Tiotropiumbromid-monohydrat: | 0,009996 mg |
| Salmeterolxinafoat | 0,043587 mg |
| Lactose Monohydrat: | 9,946417 mg |
| Polyethylen-Kapseln: | 100 mg |
| Total: | 110,0 mg |

**Beispiel 16:**

| | |
|---|---|
| Tiotropiumbromid-monohydrat: | 0,0087465 mg |
| Salmeterolxinafoat | 0,0334167 mg |
| Lactose Monohydrat: | 9,95784 mg |
| Polyethylen-Kapseln: | 100 mg |
| Total: | 110 mg |

**Beispiel 17:**

| | |
|---|---|
| Tiotropiumbromid-monohydrat: | 0,004685 mg |
| Salmeterolxinafoat | 0,03632 mg |
| Lactose Monohydrat: | 9,958995 mg |
| Polyethylen-Kanseln: | 100 mg |
| Total: | 110,0 mg |

**Beispiel 18:**

| | |
|---|---|
| Tiotropiumbromid-monohydrat: | 0,006247 mg |
| Salmeterolxinafoat | 0,03632 mg |
| Lactose Monohydrat: | 9,957433 mg |
| Polyethylen-Kapseln: | 100 ml |
| Total: | 110,0 mg |

**Beispiel 19:**

| | |
|---|---|
| Tiotropiumbromid-monohydrat: | 0,012493 mg |
| Salmeterolxinafoat | 0,03632 mg |
| Lactose Monohydrat: | 9,951187 mg |
| Polyethylen-Kanseln: | 100 mg |
| Total: | 110,0 mg |

**Darstellung der erfindungsgemäßen abgefüllten und verpackten Arzneimittel, die erfindungsgemäße Inhalationspulver enthalten:**

*Bereitstellung von vorkonditionierten Trockenmittelbeutel für die Beispiele A bis D:*

Die Konditionierung wird in einem geeigneten Klimaraum durchgeführt.

Das Trockenmittel Silicagel, Tyvekbeutel 25g bzw. 2g wird auf Hordenbleche (z.B. Hordentrocknerbleche Firma Glatt, 79589 Binzen, Deutschland) überlappungsfrei auflgelegt. Beim Einbringen der Hordenbleche in einem Hordenwagen in die Klimakammer ist zwischen den Blechen ein Abstand von mindestens 25cm zu lassen.

| Prozessdaten | Soll | Toleranzen |
|---|---|---|
| Raumklima: | 25 °C | 23 °C - 27 °C |
| | 25% r.F. | 20 % - 30 % r.F.* |
| Konditionierzeit | 96 h | 90 h - 126 h |

**Beispiel A :**

Inhalationspulver entsprechend den Beispielen 1, 5, 10 oder 13 werden auf Hordenblechen in einer Dicke von maximal 1 cm verteilt. In einem Klimaraum oder einer Klimakammer ist das Inhalationspulver mindestens 24h bei 25°C +/-2°C / 25 % +/- 3% rel. Feuchte zu konditionieren. Unter diesen klimatischen Bedingungen wird das Inhalationspulver in dicht verschließbare Stahlbehälter gefüllt.

Erfindungsgemäßes vorkonditioniertes Inhalationspulver wird direkt in einen Blisternapf gefüllt und verschlossen, wobei die Ausformung des Blisternapfes, das Abfüllen und der Versiegelungsprozess in einem klimatisiertem Arbeitsraum (22°-28°C / 20-30% rel. Feuchte) durchgeführt wird.

Derartige Blister (entspricht dem Primärpackmittel) werden zusätzlich gepoucht, wobei bei diesem Verpackungsschritt in den Pouch (entspricht dem Sekundärpackmittel) ein 2g Trockenmittelbeutel, welches bei 25% rel. Feuchte (bezogen auf 25°C) vorkonditioniertes Silicagel in einem Tyvekbeutel (Qualität: Sorb-It®, 2g, Fa. Süd Chemie AG, D-85368 Moosburg) enthält.

**Beispiel B:**

In Analogie zu der in Beispiel A beschriebenen Vorgehensweise können solche Arzneimittelblister, welche vorkonditionierte Inhalationspulver enthalten, auch direkt in Inhalationsdevices eingelegt werden. Nachfolgend ist hierbei das Inhalationsdevice zu pouchen, wobei in den Pouch (entspricht dem Sekundärpackmittel) ein 2g Trockenmittelbeutel, welches bei 25% rel. Feuchte (bezogen auf 25°C) vorkonditioniertes Silicagel in einem Tyvekbeutel (Qualität: Sorb-It®, 2g, Fa. Süd Chemie AG, D-85368 Moosburg) enthält, eingelegt wird.

**Beispiel C :**

Pulverkapseln zur Inhalation, welche gemäß den Beispielen 2 bis 5, bzw. 6-9, bzw. 11 oder 12 bzw. 14 bis 19 hergestellt werden, werden in einen dicht verschließbaren Stahlcontainer gefüllt, so dass dieser zu 30 bis maximal 75% des Füllvolumens gefüllt ist. Pro 100.000 Kapseln wird ein Trockenmittelbeutel der Qualität Sorb-It®, 25g, Fa. Süd Chemie AG, D-85368 Moosburg welches bei 25% rel. Feuchte (bezogen auf 25°C) vorkonditioniertes Silicagel in einem Tyvekbeutel enthält, in einen Einsatz in den Container überführt, so dass ein freier Gasaustausch innerhalb des Containers stattfinden kann. Der Container wird verschlossen und bei 25°C für mindestens 5 Tage zur Erreichung einer Gleichgewichtsfeuchte gelagert.

Nach Beendigung der Konditionierung der Kapseln werden diese in einem klimatisiertem Arbeitsraum (20°-28°C / 20-30% rel. Feuchte) in PE-Flaschen (entspricht dem Primärpackmittel), welche eine vorkonditionerte (20-25% reL Feuchte bei 25°C) Trockenmittelpatrone im Deckel besitzen, verpackt.

**Beispiel D:**

In Analogie zu der in Beispiel C beschriebenen Vorgehensweise können Arzneimittel, welche Inhalationskapseln enthalten, die nach der Containerkonditionierung gemäß Beispiel C konditioniert wurden, auch in erfindungsgemäße Blister (entspricht dem Primärpackmittel) verpackt werden. Die konditionierten Kapseln werden hierbei wahlweise in einem klimatisiertem Arbeitsraum (22°-28°C / 20-30% rel. Feuchte) oder einer gekapselten Verblisterungsmaschine, welche die klimatischen Bedingungen (22°-28°C / 20-30% rel. Feuchte) einhält, verblistert. Hierbei finden Blisterfolien Einsatz, welche vorzugsweise mindestens 12 Monate bei 20°-28°C / 20-30% rel. Feuchte aufbewahrt wurden.

Derartige Blister werden zusätzlich gepoucht, wobei bei diesem Verpackungsschritt in den Pouch (entspricht dem Sekundärpackmittel) ein 2g Trockenmittelbeutel, welches bei 25% rel. Feuchte (bezogen auf 25°C) vorkonditioniertes Silicagel in einem Tyvekbeutel (Qualität: Sorb-It®, 2g, Fa. Süd Chemie AG, D-85368 Moosburg) enthält, eingelegt wird.

**III) Beispiele zur Darstellung der Eignung erfindungsgemäßer Inhalationspulver als inhalatives Arzneimittel**

Die Darstellung der besonderen Eignung der erfindungsgemäßen Inhalationspulver ist beispielsweise dahingehend möglich, indem diese auf ihre inhalative Performance sowie deren Zersetzungsrate des Wirkstoffes als Funktion der Zeit getestet werden.

Die inhalative Performance ist dabei bestimmbar, indem die Fine Particle Fraxction (FPF) des Arzneimittels analysiert wird. Unter der FPF ist die inhalierbare Dosis (Partikel < 5 μm) zu verstehen, die auf Basis der Pharm. Eur. 2.9.18 (European Pharmacopoeia, 6th edition 2008, Apparatus D - Andersen Cascade Impactor) bzw. USP30-NF25 <601> bestimmbar ist, und in relativem Bezug zu der nominalen Wirkstoffdosis der getesteten Menge (Dosis) gesetzt wird. Das Ergebnis ist folglich in % anzugeben.

Erfindungsgemäß erfolgte die Testung (gemäß folgenden Beispielen I und II) anhand von Inhalationspulver, die nach Beispiel 14 hergestellt wurden und mit einem Inhalator entsprechend Figur 2 ausgebracht wurden.

**Beispiel I**

Ergebnisse der FPF (Tiotropium) von 4 Chargen sind in Figur 3 dargestellt. Von jeder der vier Chargen wurden Inhalationskapseln, wie sie nach Beispiel 14 erhältlich sind, einerseits direkt nach der Herstellung der Kapseln verpackt

und andererseits. in ein Gleichgewicht mit einer Umgebungsfeuchte gebracht, so dass der $a_w$-Wert, als Kenngröße des Inhalationspulvers kleiner 0.05 (bezogen auf 25°C) war. Das Muster, welches nach Herstellung ohne weitere Nachbehandlung analysiert wurde" befand sich in einem Gleichgewicht mit einem $a_w$-Wert zwischen 0.45 und maximal 0.55 (bezogen auf 25°C).

Dabei zeigt sich, dass Inhalationspulver, enthaltend ein Salmeterolsalz und ein Tiotropiumsalz, und die im Gleichgewicht zu einem $a_W$-Wert kleiner 0.05 stehen, eine niedrigere FPF aufweisen, als wenn sie im Gleichgewicht zu einem $a_W$-Wert zwischen 0.45 und 0.55 (bezogen auf 25°C) gebracht sind. Die FPF (Tiotropium) für das Produkt, welches gekennzeichnet ist durch einen $a_W$-Wert von 0.45 - 0.55 beträgt mehr als 30%. Die FPF für das Produkt, welches gekennzeichnet ist durch einen $a_W$-Wert von kleiner 0.05 ist kleiner 30%.

Ein Rückgang / Differenz der FPF ist um 20% (bezogen auf den höheren FPF Wert) festzustellen.

**Beispiel II**

Ergebnisse der FPF von Salmeterol und von Tiotropium einer Charge, wie sie nach Beispiel 14 erhältlich sind, sind in Figur 4 dargestellt. Die vordere Säulenreihe entspricht der FPF des Tiotropiums, die hintere Säulenreihe der des Salmeterols. Das Produkt wurde initial auf eine Gleichgewichtsfeuchte gebracht, so dass der $a_W$-Wert zwischen 0.28 (bezogen auf 25°C) betrag. Die initialen FPF für Tiotropium und Salmeterol dieser Charge entsprechen einem $a_w$-Wert von 0.28. Zur Überprüfung der Stabilität wurde das Produkt Bedingungen ausgesetzt, so dass das Inhalationspulver in den Kapseln im Gleichgewicht einer Umgebungsfeuchte stand, die einem $a_W$-Wert von 0.1, bzw. 0.3 bzw. 0.4 bzw. 0.6 entsprechen (bezogen auf 25°C).

Der initiale FPF des Salmeterols ($a_W$-Wert = 0.28) betrug ca. 40%. Der FPF des Salmeterols ($a_W$-Wert = 0.1, 0.3 und 0.4) betrug nach 18 Monaten Lagerung mehr als 30 %. Der initiale FPF des Tiotropiums ($a_W$-Wert = 0.28) betrug ca. 30%. Der FPF des Tiotropiums ($a_w$-Wert = 0.1, 0.3 und 0.4) betrug nach 18 Monaten Lagerung mehr als 25%. Der FPF des Salmeterols ($a_W$-Wert = 0.6) betrug nach 18 Monaten Lagerung weniger als 30%. Der FPF des Tiotropiumss ($a_w$-Wert = 0.6) betrug nach 18 Monaten Lagerung weniger als 25%.

| | FPF [%] | | | | |
|---|---|---|---|---|---|
| | initial | aW=0,1 (18 Monate) | aW=0,3 (18 Monate) | aW=0,4 (18 Monate) | aW=0,6 (18 Monate) |
| **Wert FPF[%] - Sal** | 40 | 37 | 38 | 35 | 27 |
| **Wert FPF[%] - Tio** | 31 | 30 | 31 | 31 | 23 |

Während hingegen die FPF für Tiotropium wie auch Salmeterol nach 18 Monaten vergleichbar zum initialen Wert ist, ist eine Abnahme derselben von mehr als 25% (Tiotropium) bzw. mehr als 30% (Salmeterol) beobachtbar (jeweils bezogen auf initialen Wert).

**Beispiel III**

Ergebnisse des Zersetzungsverlaufs von Salmeterol einer Charge, wie sie nach Beispiel 14 erhältlich sind, sind in Figur 5 dargestellt. Zur Überprüfung der Stabilität wurde das Produkt Bedingungen ausgesetzt, so dass das Inhalationspulver in den Kapseln im Gleichgewicht einer Umgebungsfeuchte stand, die einem $a_w$-Wert von 0.1, bzw. 0.3 bzw. 0.4 bzw. 0.6 entsprechen (bezogen auf 25°C). Es zeigt sich dabei, dass ein akzeptabler Zersetzungsverlauf über einen Zeitraum von mindestens 18 Monaten nur dann gewährleistet werden kann, wenn das Produkt im Gleichgewicht mit der Feuchte (gemessen direkt über dem Produkt) gehalten wird, der einem $a_W$-Wert von 0.1 oder 0.3 oder 0.4 entspricht.

**Patentansprüche**

1. Verfahren zur Bereitstellung von Inhalationspulver enthaltend Laktose, ein Salmeterolsalz und ein Tiotropiumsalz **dadurch gekennzeichnet, dass** das Inhalationspulver einer Feuchte ausgesetzt wird, so dass sich über dem Inhalationspulver ein aw-Wert bei 25°C zwischen 0.1 und 0.4 im Gleichgewicht einstellt, **dadurch gekennzeichnet, dass** die Inhalationspulver

- mind. 4 Stunden bei einer rel. Feuchte von 18 bis 27% und 16°C bis 28°C konditioniert werden
- darauf folgend die vordosierten Inhalationspulver in einer Umgebungsfeuchte von 20 bis 30% und einer Temperatur von 23 bis 28°C verpackt werden.

**2.** Verfahren nach Anspruch 1, **dadurch gekennzeichnet, dass** zur Verpackung der vordosierten Inhalationspulver ein Packmaterial verwendet wird, welches **gekennzeichnet ist durch** eine Permeationsrate für Wasser von weniger als 5g/m $^2$d.

**3.** Verfahren zur Bereitstellung von verpackten Arzneimitteln zur Inhalation nach einem der Ansprüche 1 oder 2, wobei dieses die Schritte

- Bereitstellung von vordosierten Inhalationspulvern enthaltend Laktose, ein Salmeterolsalz und ein Tiotropiumsalz **dadurch gekennzeichnet, dass** das Inhalationspulver einer Feuchte ausgesetzt wird, so dass sich über dem Inhalationspulver ein aw-Wert bei 25°C zwischen 0.1 und 0.4 im Gleichgewicht einstellt und
- darauf folgend primar-verpackte Inhalationspulver in einer Umgebungsfeuchte von 20 bis 30% und einer Temperatur von 23 bis 28°C sekundär verpackt werden.

**4.** Verfahren nach Anspruch 3, **dadurch gekennzeichnet, dass** ein vorbefeuchtete Trockenmittel in das Sekundarpackmittel zusammen mit dem primär verpackten, vordosiertem Inhalationspulver eingelegt wird.

## Claims

**1.** Method for preparing inhalation powders containing lactose, a salmeterol salt and a tiotropium salt, **characterised in that** the inhalation powder is exposed to a humidity such that at 25°C an $a_W$ value between 0.1 and 0.4 is in equilibrium above the inhalation powder, **characterised in that** the inhalation powders

- are conditioned for at least 4 hours at a relative humidity of 18 to 27% and at 16°C to 28°C
- after which the pre-dosed inhalation powders are packaged in an ambient humidity of 20 to 30% and at a temperature of 23 to 28°C.

**2.** Method according to claim 1, **characterised in that** for the packaging of the pre-dosed inhalation powders a packaging material is used which is **characterised by** a permeation rate for water of less than 5 g/m$^2$d.

**3.** Method for preparing packaged medicaments for inhalation according to one of claims 1 or 2, comprising the steps of

- preparing pre-dosed inhalation powders containing lactose, a salmeterol salt and a tiotropium salt, **characterised in that** the inhalation powder is exposed to a humidity such that at 25°C an $a_W$ value between 0.1 and 0.4 is in equilibrium above the inhalation powder and
- subsequently inhalation powders in their primary packaging are provided with a secondary packaging in an ambient humidity of 20 to 30% and at a temperature of 23 to 28°C.

**4.** Method according to claim 3, **characterised in that** a pre-moistened desiccant is placed in the secondary packaging means together with the pre-dosed inhalation powder in its primary packaging.

## Revendications

**1.** Procédé pour fournir une poudre pour inhalation contenant du lactose, un sel de salmétérol et un sel de tiotropium, **caractérisé en ce que** la poudre pour inhalation est exposée à une humidité, de sorte qu'il s'instaure sur la poudre pour inhalation, une valeur aw à 25 °C entre 0,1 et 0,4 à l'état d'équilibre, **caractérisé en ce que** les poudres pour inhalation

- sont conditionnées pendant au moins 4 heures à une humidité relative de 18 à 27 % et 16 °C à 28 °C
- ensuite, les poudres pour inhalation prédosées sont empaquetées à une humidité ambiante de 20 à 30 % et à une température de 23 à 28 °C.

**2.** Procédé selon la revendication 1, **caractérisé en ce que**, pour l'empaquetage de la poudre pour inhalation prédosée,

on utilise un matériau d'emballage qui est **caractérisé par** un taux de perméation de l'eau inférieur à 5 g/m$^2$d.

**3.** Procédé pour fournir des médicaments empaquetés pour inhalation selon l'une des revendications 1 ou 2, celui-ci comprenant les étapes de

- fourniture de poudres pour inhalation prédosées contenant du lactose, un sel de salmétérol et un sel de tiotropium, **caractérisé en ce que** la poudre pour inhalation est exposée à une humidité, de sorte qu'il s'instaure sur la poudre pour inhalation, une valeur aw à 25 °C entre 0,1 et 0,4 à l'état d'équilibre et
- ensuite, des poudres pour inhalation empaquetées dans un emballage primaire sont empaquetées dans un emballage secondaire à une humidité ambiante de 20 à 30 % et à une température de 23 à 28 °C.

**4.** Procédé selon la revendication 3, **caractérisé en ce qu'**un dessicatif préhumidifié est placé dans l'emballage secondaire conjointement avec la poudre pour inhalation prédosée empaquetée dans un emballage primaire.

Figur 1:

**Röntgenpulverdiagramm**

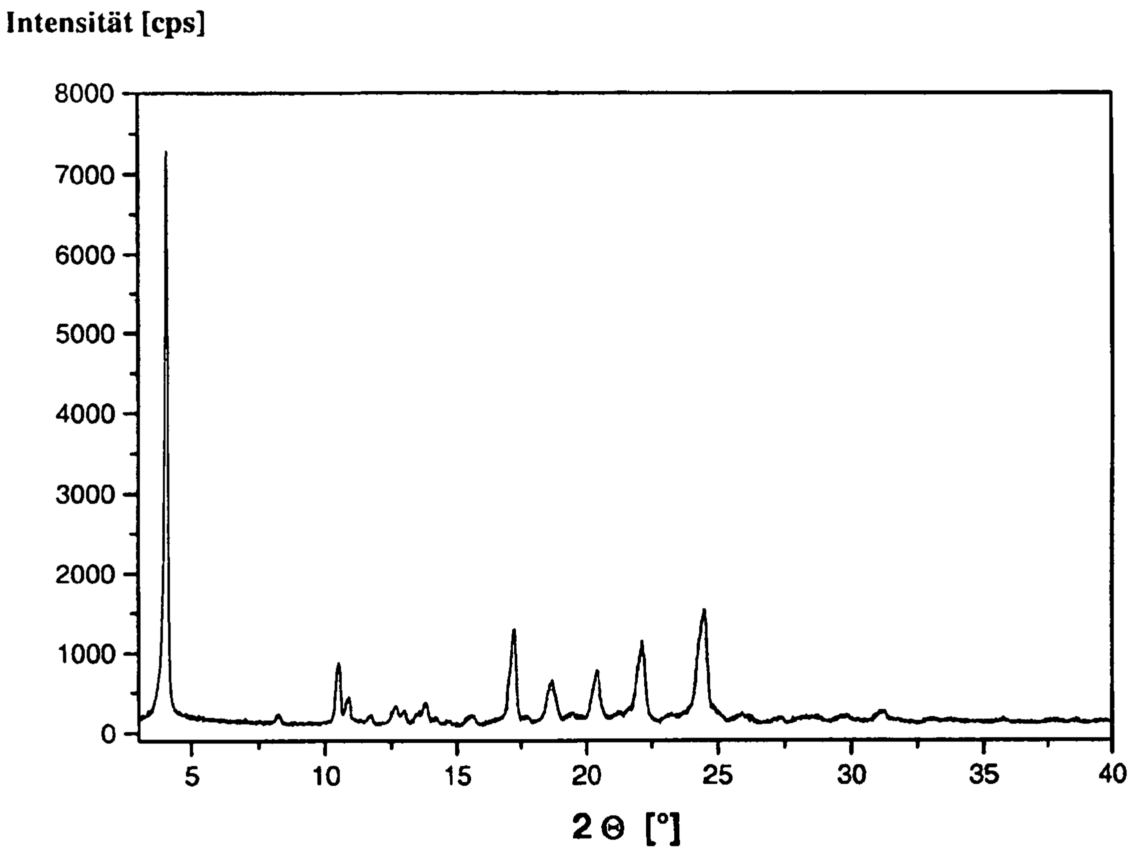

Figur 2:

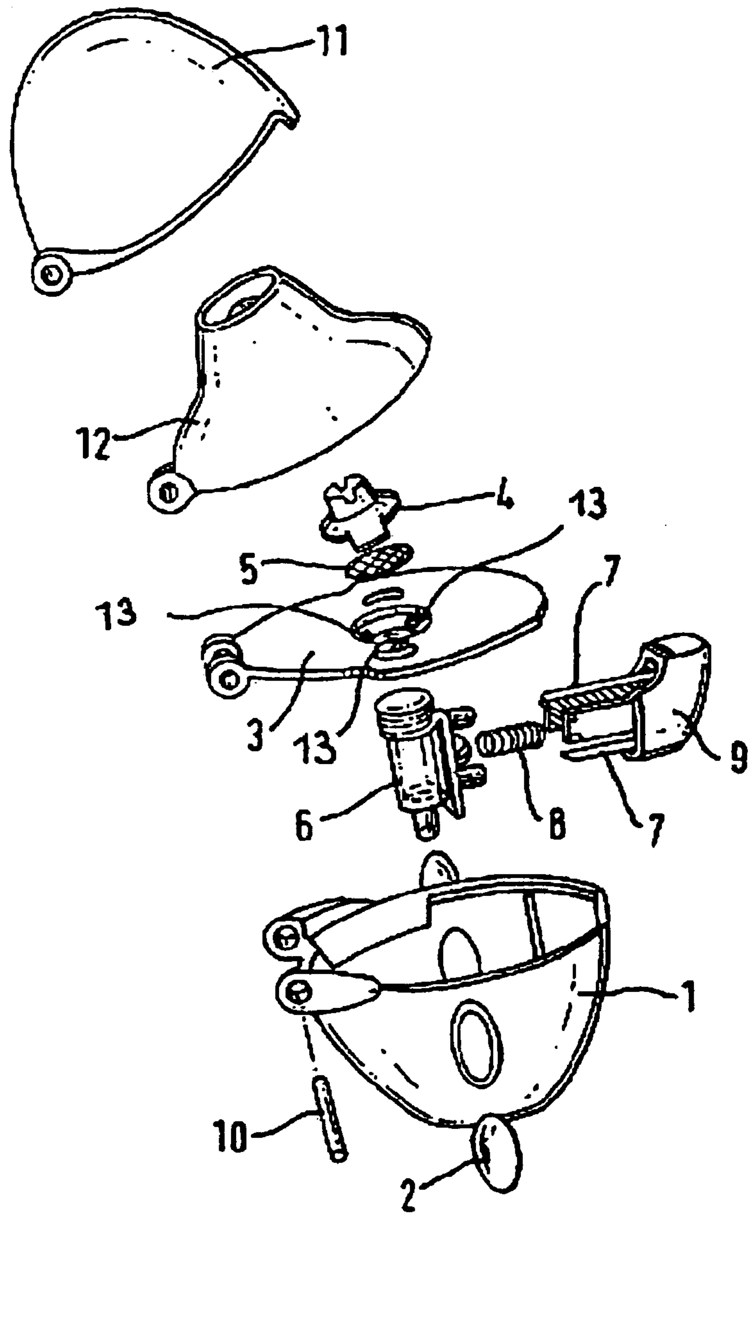

Figur 3:

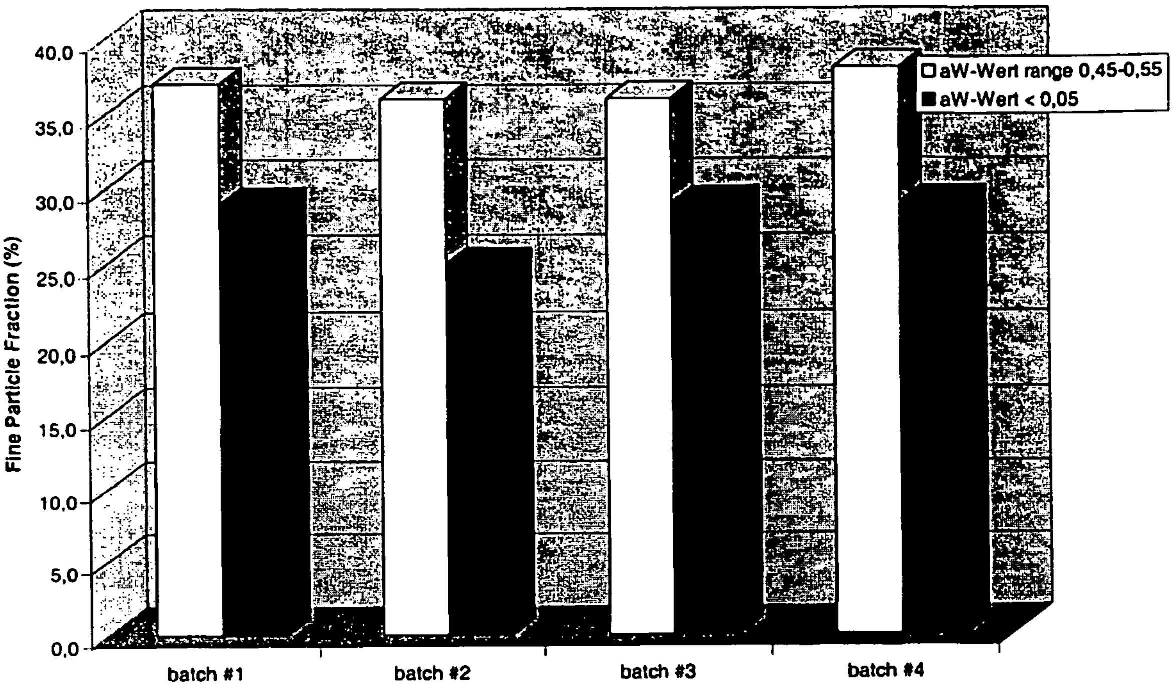


Figur 4:

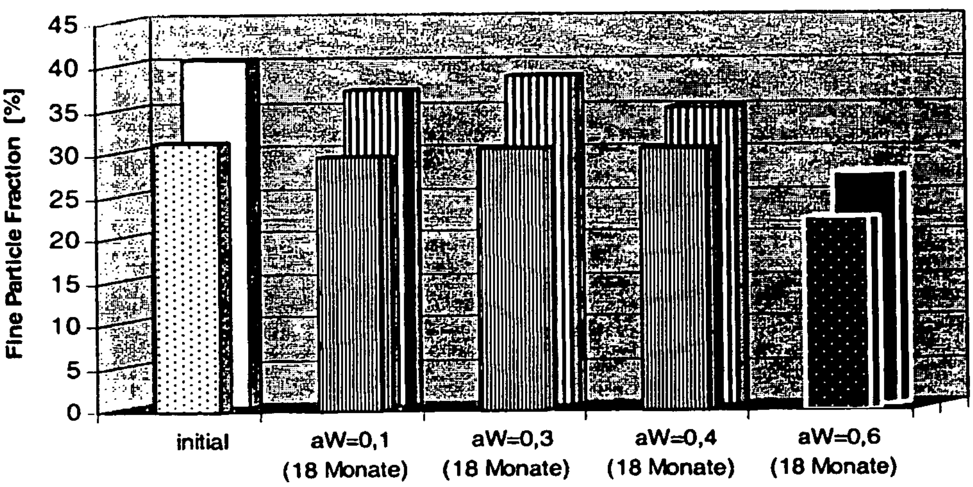

Figur 5:

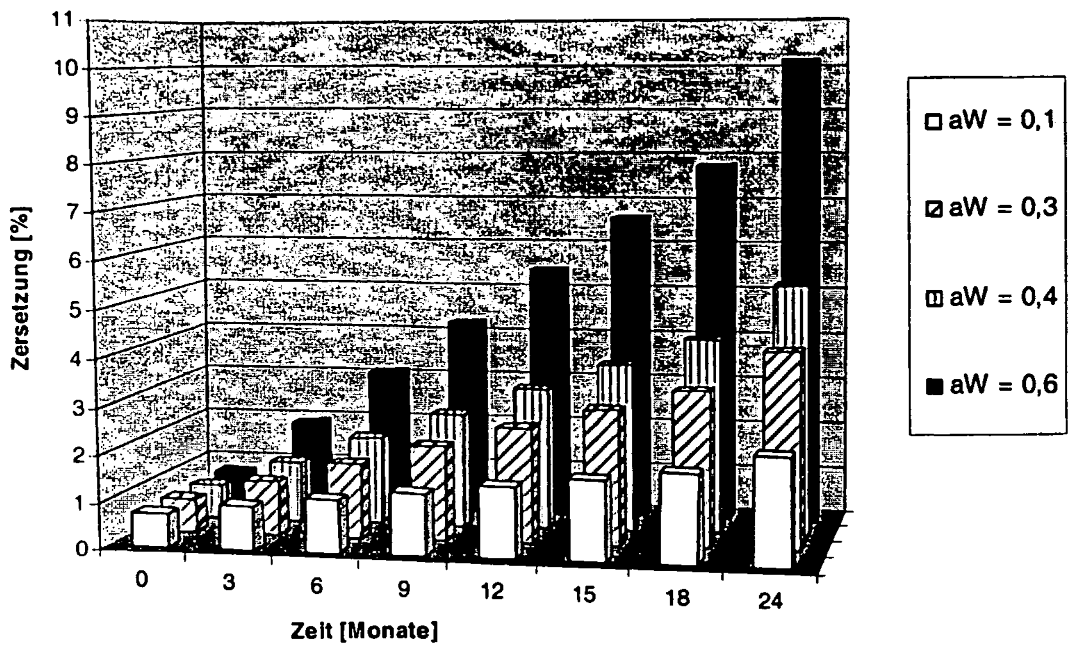
11
10
9
8
7
6
5
4
3
2
1
0
Zersetzung [%]
0
3
6
9
12
15
18
24
Zeit [Monate]
aW = 0,1
aW = 0,3
aW = 0,4
aW = 0,6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 418716 A1
- WO 0069468 A
- WO 20040582 A
- DE 1792207 A
- EP 0479282 A1
- WO 2004105727 A2
- WO 0230928 A
- WO 0230390 A
- WO 2004058233 A


### In der Beschreibung aufgeführte Nicht-Patentliteratur


- **XU, H. ; TEMPLETON, A.C. ; ZWIERZYNSKI, M. ; MAHAJN R. ; REED, R.A.** Rapid, simultaneous determination of headspace oxygen and moisture in pharmaceutical packages using μGC. *Journal of Pharmaceutical and Biomedical Anaysis,* 2005, vol. 38, 225-231
- Apparatus D - Andersen Cascade Impactor. Pharm. Eur. 2.9.18. 2008